# EUROPÄISCHE PATENTSCHRIFT

(11) **EP 0 877 018 B1**
(45) Veröffentlichungstag und Bekanntmachung des Hinweises auf die Patenterteilung: **02.05.2003**
(21) Anmeldenummer: 98108038.5
(22) Anmeldetag: 02.05.1998
(51) Int. Cl.: C07C 311/19, C07C 311/29, A61K 31/18, A61K 31/63, C07D 207/16, C07D 409/04, C07D 413/04, C07D 277/16, C07D 333/24, C07D 333/60, C07D 209/18, C07D 233/54, C07D 317/60, C07D 213/42, C07D 205/04, C07D 263/06, C07D 211/34, C07D 401/12, C07D 203/24, C07D 249/06, C07D 403/12, C07D 261/08, C07D 295/10, C07D 235/16, C07D 209/08

(54) **Sulfonylaminocarbonsäuren**
Sulfonylaminocarboxylic acids
Acides sulfonylaminocarboxyliques

(30) Priorität: 09.05.1997 DE 19719621
(43) Veröffentlichungstag der Anmeldung: 11.11.1998
(73) Patentinhaber: Aventis Pharma Deutschland GmbH, 65929 Frankfurt am Main (DE)
(72) Erfinder: Thorwart, Werner, Dr., 65239 Hochheim (DE); Schwab, Wilfried, Dr., 65193 Wiesbaden (DE); Schudok, Manfred, Dr., 65817 Eppstein/Ts. (DE); Haase, Burkhard, Dr., 65719 Hofheim (DE)

(56) Entgegenhaltungen:
- EP-A- 0 606 046
- EP-A- 0 757 037
- EP-A- 0 950 656
- EP-B- 0 305 947
- EP-B- 0 468 231
- WO-A-95/35276
- WO-A-96/00214
- WO-A-96/27583
- WO-A-96/33172
- WO-A-97/19068
- WO-A-97/44315
- R.P. Beckett et al., DDT Vol.1, No.1, 1996, pp. 16-26
- J.Med. Chem. 1998, 41, 640-649
- J.Org.Chem. 1988, 53, 2367-71

## Beschreibung

Die Erfindung betrifft neue Sulfonylaminocarbonsäuren, Verfahren zu ihrer Herstellung und Verwendung derselben als Arzneimittel.

In den Anmeldungen EP 0 606 046, WO 95/35276 und WO 96/27583 werden Arylsulfonaminohydroxamsäuren und deren Wirkung als Matrix-Metallproteinase-Inhibitoren beschrieben. Spezielle Arylsulfonaminocarbonsäuren, dienen als Zwischenprodukte zur Darstellung von Thrombin-Inhibitoren (EP 0 468 231) und Aldose-Reduktase-Inhibitoren (EP 0 305 947). In der Anmeldung EP 0 757 037 wird auch die Wirkung von Sulfonylaminocarbonsäure-Derivate als Metalloproteinase-Inhibitoren beschrieben.

Ferner hat sich die Arylsulfonylgruppe als eine effektive Schutzgruppe der Aminofunktion von α-Aminocarbonsäuren bewährt (R. Roemmele, H. Rapoport, J. Org. Chem. 53 (1988) 2367-2371).

Die Dokumente WO 97/27174 und WO 97/44315 beschreiben Verbindungen, welche die Wirkung von Matrix Metalloproteinasen inhibieren können. Diese beiden Dokumente haben einen Anmeldetag, der vor dem Anmeldetag der vorliegenden Patentanmeldung liegt, aber erst nach diesem Anmeldetag publiziert wurden.

In dem Bestreben wirksame Verbindungen zur Behandlung von Bindegewebserkrankungen zu finden, wurde nun gefunden, daß die erfindungsgemäßen Sulfonylaminocarbonsäuren starke Inhibitoren der Matrix-Metalloproteinasen sind. Dabei wird auf die Hemmung von Stromelysin (Matrix Metalloproteinase 3) und der Neutrophilen Kollagenase (MMP-8) besonderer Wert gelegt, da beide Enzyme beim Abbau der Proteoglykane, als wichtige Bestandteile des Knorpelgewebes, maßgeblich beteiligt sind (A. J. Fosang et al. J. Clin. Invest. 98 (1996) 2292-2299).

Die Erfindung betrifft daher die Verbindung der Formel I 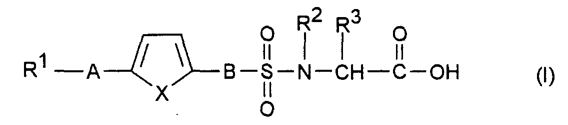 und/oder eine stereoisomere Form der Verbindung der Formel 1 und/oder ein physiologisch verträgliches Salz der Verbindung der Formel 1, wobei
R¹ für
   1. Phenyl,
   2. Phenyl, welches ein- oder zweifach substituiert ist durch
      2.1. (C₁-C₆)-Alkyl, gerade, cyclisch oder verzweigt,
      2.2. -OH,
      2.3. (C₁-C₆)-Alkyl-C(O)-O-,
      2.4. (C₁-C₆)-Alkyl-O-,
      2.5. (C₁-C₆)-Alkyl-O-(C₁-C₄)-Alkyl-O-,
      2.6. Halogen,
      2.7. -CF₃,
      2.8. -CN,
      2.9. -NO₂,
      2.10. HO-C(O)-,
      2.11. (C₁-C₆)-Alkyl-O-C(O)-,
      2.12. Methylendioxo,
      2.13. R⁴-(R⁵)N-C(O)- oder
      2.14. R⁴-(R⁵)N-, oder
   3. einen Heteroaromaten aus der nachfolgenden Gruppe 3.1. bis 3.15., der unsubstituiert oder wie unter 2.1 bis 2.14 beschrieben substituiert ist,
      3.1. Pyrrol,
      3.2. Pyrazol,
      3.3. Imidazol,
      3.4. Triazol,
      3.5. Thiophen,
      3.6. Thiazol,
      3.7. Oxazol,
      3.8. Isoxazol,
      3.9. Pyridin,
      3.10. Pyrimidin,
      3.11. Indol,
      3.12 Benzothiophen,
      3.13. Benzimidazol,
      3.14. Benzoxazol oder
      3.15. Benzothiazol steht,
R², R⁴ und R⁵ gleich oder verschieden sind und für
   1. Wasserstoffatom,
   2. (C₁-C₆)-Alkyl-,
   3. HO-C(O)-(C₁-C₆)-Alkyl-,
   4. Phenyl-(CH₂)ₙ-, wobei Phenyl unsubstituiert oder ein- oder zweifach wie unter 2.1 bis 2.14 beschrieben substituiert ist oder durch -NH-C(O)-(C₁-C₃)-Alkyl substituiert ist substituiert ist und n die ganze Zahl Null, 1 oder 2 darstellt, oder
   5. Picolyl stehen oder
   6. R⁴ und R⁵ zusammen mit der ringständigen Aminogruppe einen 4- bis 7-gliedrigen Ring bilden, worin gegebenenfalls eines der Kohlenstoffatome durch -O-, -S- oder -NH- ersetzt ist oder zwei benachbarte C-Atome des 4-bis 7-gliedrigen Rings Teil eines Benzylrestes sind,
R³ für
   1. (C₂-C₁₀)-Alkenyl-, worin Alkenyl gerade oder verzweigt ist,
   2. R²-S(O)ₙ-(C₁-C₆)-Alkyl-, wobei n die obengenannte Bedeutung hat und worin R² die obengenannte Bedeutung für 2. bis 5. hat,
   3. R²-S(O)(=NH)-(C₁-C₆)-Alkyl-, worin R² die obengenannte Bedeutung für 2. bis 5. hat,
   4. 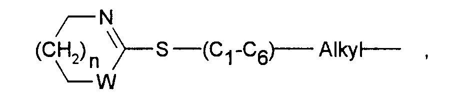 worin n die ganze Zahl Null, 1 oder 2 darstellt und W ein Stickstoff-, Sauerstoff- oder Schwefelatom darstellt,
   5. -(CH₂)ₘ-P(O)(OH)-(C₁-C₃)-Alkyl, worin m die ganze Zahl Null, 1, 2, 3, 4, 5 oder 6 darstellt, oder
   6. R⁶-C(O)-(C₁-C₆)-Alkyl- steht, worin R⁶ für
      1. (C₁-C₆)-Alkyl-,
      2. HO-C(O)-CH(R³)-NH-, worin R³ für
         2.1. Wasserstoffatom,
         2.2. (C₁-C₁₀)-Alkyl-, worin Alkyl unsubstituiert ist, und/oder ein Wasserstoffatom des Alkylrestes durch -OH ersetzt ist,
         2.3. (C₂-C₁₀)-Alkenyl-, worin Alkenyl gerade oder verzweigt ist,
         2.4. R²-O-(C₁-C₆)-Alkyl-, worin R² die obengenannte Bedeutung für 1. bis 5. hat,
         2.5. R²-S(O)ₙ-(C₁-C₆)-Alkyl-, wobei n die ganze Zahl Null, 1 oder 2 darstellt und worin R² die obengenannte Bedeutung für 1. bis 5. hat,
         2.6. R²-S(O)(=NH)-(C₁-C₆)-Alkyl-, worin R² die obengenannte Bedeutung für 1. bis 5. hat,
         2.7. 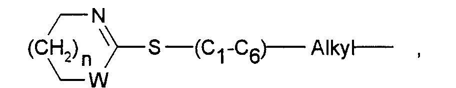 worin n die ganze Zahl Null, 1 oder 2 darstellt und W ein Stickstoff-, Sauerstoff- oder Schwefelatom darstellt,
         2.8. Phenyl-(CH₂)ₘ-, worin m die ganze Zahl Null, 1, 2, 3, 4, 5 oder 6 ist und/oder ein Wasserstoffatom der -(CH₂)ₘ-Kette durch -OH ersetzt ist und Phenyl unsubstituiert oder ein- oder zweifach substituiert ist mit
            2.8.1 wie oben unter 2.1. bis 2.14. beschrieben,
            2.8.2 -O-(CH₂)ₘ-Phenyl, worin Phenyl unsubstituiert oder ein- oder zweifach wie oben unter 2.1. bis 2.14. beschrieben substituiert ist und m die ganze Zahl Null, 1, 2, 3, 4, 5 oder 6 darstellt, oder
            2.8.3 -C(O)-(CH₂)ₘ-Phenyl, worin Phenyl und m wie oben unter 2.8.2 definiert ist,
         2.9. Heteroaryl-(CH₂)ₘ-, worin Heteroaryl wie unter 3.1. bis 3.15. definiert ist, m die ganze Zahl Null, 1, 2, 3, 4, 5 oder 6 darstellt wie oben definiert ist und/oder ein Wasserstoffatom der -(CH₂)ₘ-Kette durch -OH ersetzt ist und Heteroaryl unsubstituiert oder ein- oder zweifach substituiert ist mit
            2.9.1 wie oben unter 2.1 bis 2.14 beschrieben,
            2.9.2 -CH(O),
            2.9.3 -SO₂-Phenyl, worin Phenyl unsubstituiert oder wie oben unter 2.8.2 oder 2.8.3 definiert ist, oder
            2.9.4 -O-(CH₂)ₘ-Phenyl, worin m die ganze Zahl Null, 1, 2, 3, 4, 5 oder 6 ist, oder
         2.10. -(CH₂)ₘ-P(O)(OH)-(C₁-C₃)-Alkyl, worin m die ganze Zahl Null, 1, 2, 3, 4, 5 oder 6 ist,
      3. Phenyl, worin Phenyl unsubstituiert oder wie unter 2.1. bis 2.14. 2.14.beschrieben substituiert ist,
      4. Heteroaryl, worin Heteroaryl wie unter 3.1. bis 3.15. definiert und/oder wie unter 2.1 bis 2.14 beschrieben substituiert ist,
      5. R⁴-(R⁵)N-NH-, worin R⁴ und R⁵ wie oben definiert sind,
      6. R²O-, worin R² die obengenannte Bedeutung für 2. bis 5. hat,
      7. R⁴-(R⁵)N- bedeutet, worin R⁴ und R⁵ die obengenannte Bedeutung für 1. bis 5. haben, aber nicht gleichzeitig Wasserstoffatom sind, oder
      8. Heteroaryl-(CH₂)ₘ-NH-, worin Heteroaryl wie unter 3.1. bis 3.15. definiert und/oder wie unter 2.1. bis 2.14 beschrieben substituiert ist und m die ganze Zahl Null, 1, 2, 3, 4, 5 oder 6 darstellt oder durch -(C₁-C₄)-Alkyl-COOH substituiert ist, steht, oder
R² und R³ bilden zusammen einen Ring mit ringständiger Carboxylgruppe der Teilformel II, 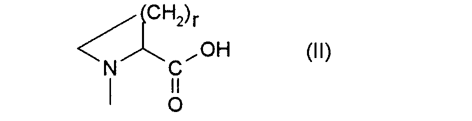 worin r die ganze Zahl Null, 1, 2 oder 3 darstellt und/oder eines der Kohlenstoffatome im Ring durch -O-, -S- oder -(R⁷)N- ersetzt ist, worin
R⁷ für
   1. Wasserstoffatom,
   2. (C₁-C₆)-Alkyl,
   3. Phenyl, worin Phenyl unsubstituiert oder wie unter 2.1 bis 2.14 beschrieben substituiert ist,
   4. Benzyl, worin Benzyl unsubstituiert oder wie unter 2.1 bis 2.14 beschrieben substituiert ist, oder
   5. R²N-C(=NH)- steht, wobei R² die obengenannte Bedeutung für 1. bis 5. hat,
und/oder die Kohlenstoffatome im Ring der Teilformel II ein- oder mehrfach durch (C₁-C₆)-Alkyl-, Phenyl-, Phenyl-(CH₂)ₘ- oder -OH substituiert sind, worin m die ganze Zahl Null, 1, 2, 3, 4, 5 oder 6 darstellt,
A für
   a) eine kovalente Bindung,
   b) -O-,
   c) -CH=CH- oder
   d) -C≡C- steht,
B für
   a) -(CH₂)ₘ-, worin m die ganze Zahl Null, 1, 2, 3, 4, 5 oder 6 darstellt,
   b) -O-(CH₂)ₚ, worin p eine ganze Zahl von 1 bis 5 bedeutet, oder
   c) -CH=CH- steht und
X für -CH=CH-, Sauerstoffatom oder Schwefelatom steht.

Bevorzugt ist eine Verbindung der Formel I, wobei
R¹ für
   1. Phenyl oder
   2. Phenyl, welches einfach substituiert ist durch
      2.1. (C₁-C₆)-Alkyl-, worin Alkyl gerade, cyclisch oder verzweigt ist,
      2.2. -OH,
      2.3. (C₁-C₆)-Alkyl-C(O)-O-,
      2.4. (C₁-C₆)-Alkyl-O-,
      2.5. (C₁-C₆)-Alkyl-O-(C₁-C₄)-Alkyl-O-,
      2.6. Halogen,
      2.7. -CF₃ oder
      2.8. R⁴-(R⁵ )N- steht,
R², R⁴ und R⁵ gleich oder verschieden sind und für
   1. Wasserstoffatom oder
   2. (C₁-C₆)-Alkyl- stehen,
R³ für
   1. R²-S(O)ₙ-(C₁-C₆)-Alkyl-, worin R² (C₁-C₆)-Alkyl- oder Phenyl-(CH₂)ₙ- bedeutet und n die ganze Zahl Null oder 1 ist, oder
   2. R⁶-C(O)-(C₁-C₆)-Alkyl- steht, worin
      R⁶ für
      1. R²O-, worin R² Wasserstoffatom oder (C₁-C₆)-Alkylbedeutet, oder
      2. R⁴-(R⁵)N- steht, worin R⁴ und R⁵ die obengenannte Bedeutung für 1. bis 2. haben, aber nicht gleichzeitig Wasserstoffatom sind, oder R⁴ und R⁵ zusammen mit der ringständigen Aminogruppe einen 5- bis 6-gliedrigen Ring bilden, worin gegebenenfalls eines der Kohlenstoffatome durch -O-, -S- oder -NH- ersetzt ist oder zwei benachbarte C-Atome des 5- bis 6- gliedrigen Rings Teil eines Benzylrestes sind, oder
R² und R³ bilden zusammen einen Ring mit ringständiger Carboxylgruppe der Teilformel II, worin n die ganze Zahl 1 oder 2 bedeutet und/oder eines der Kohlenstoffatome im Ring durch -O- oder -(R⁷)N- ersetzt ist, und
   R⁷ für
      1. Wasserstoffatom,
      2. (C₁-C₆)-Alkyl,
      3. Phenyl, worin Phenyl unsubstituiert oder wie oben 2.1 bis 2.14 beschrieben substituiert ist,
      4. Benzyl, worin Benzyl unsubstituiert oder wie oben 2.1 bis 2.14 beschrieben substituiert ist, oder
      5. R²N-C(=NH)- steht, worin R² (C₁-C₆)-Alkyl- bedeutet, und/oder die Kohlenstoffatome im Ring der Teilformel II mit Phenyl oder -OH einfach substituiert sind,
A für
   a) eine kovalente Bindung oder
   b) -O- steht,
B für
   a) -(CH₂)ₘ-, worin m die ganze Zahl Null, 1 oder 2 bedeutet, oder
   b) -O-(CH₂)ₚ, worin p eine ganze Zahl von 1 oder 2 bedeutet, steht und
X für -CH=CH- steht.

Besonders bevorzugt ist eine Verbindung der Formel I, wobei
R¹ für
   1. Phenyl oder
   2. Phenyl, welches einfach substituiert ist durch
      2.1. Halogen, insbesondere Chlor oder Fluor oder
      2.2. R⁴-(R⁵)N-, wobei R⁴ und R⁵ gleich oder verschieden sind und für
         2.2.1. (C₁-C₃)-Alkyl oder
         2.2.2. R⁴ und R⁵ zusammen mit der ringständigen Aminogruppe einen 5-6-gliedrigen Ring bilden, wobei gegebenenfalls eines der C-Atome durch -O- oder -N- ersetzt ist, stehen,
R² für Wasserstoffatom steht,
R³ für 1.
   R⁶-C(O)-(C₂-C₃)-Alkyl steht, worin
   R⁶ für 1. R⁴-(R⁵)N- steht, worin R⁴ und R⁵ gleich oder verschieden sind, aber nicht gleichzeitig Wasserstoffatom sind und für
      1.1. Wasserstoffatom,
      1.2. (C₁-C₃)-Alkyl-,
      1.3. Phenyl-(CH₂)ₙ-, wobei Phenyl unsubstituiert oder ein- oder zweifach wie unter 2.1 bis 2.14 beschrieben substituiert ist und n die ganze Zahl Null, 1, oder 2 darstellt,
      1.4. R⁴ und R⁵ zusammen mit der ringständigen Aminogruppe einen 5-6-gliedrigen Ring bilden, wobei gegebenenfalls eines der C-Atome durch -O- oder -NH- ersetzt ist oder einen Indolin-Rest bilden, oder
      1.5. HO-C(O)-CH(R³)-NH-, worin R³ Heteroaryl-(CH₂)ₘ-, worin Heteroaryl wie oben unter 3.5, 3.11 oder 3.13 definiert ist und das Heteroaryl unsubstituiert oder einfach substituiert ist wie unter 2.1 bis 2.14 beschrieben und m die ganze Zahl 1 oder 2 bedeutet,
A für eine kovalente Bindung steht,
B für -(CH₂)ₒ- steht, worin o Null bedeutet und
X für -CH=CH- steht.

Mit dem Begriff "R⁴ und R⁵ zusammen mit der ringständigen Aminogruppe einen 4- bis 7-gliedrigen Ring bilden und/oder eines der Kohlenstoffatome durch -O-, -S- oder -NHersetzt ist" werden Reste verstanden, die sich beispielsweise von Azetidin, Pyrrol, Pyrrolin, Pyridin, Azepin, Piperidin, Oxazol, Isoxazol, Imidazol, Indolin, Pyrazol, Thiazol, Isothiazol, Diazepin, Thiomorpholin, Pyrimidin oder Pyrazin ableiten. Unter dem Begriff "Halogen" wird Fluor, Chlor, Brom oder Jod verstanden. Unter dem Begriff "Alkyl" oder "Alkenyl" werden Kohlenwasserstoffreste verstanden deren Kohlenstoffketten geradkettig oder verzweigt sind. Cyclische Alkylreste sind beispielsweise 3- bis 6-gliedrige Monocyclen wie Cyclopropyl, Cyclobutyl, Cyclopentyl oder Cyclohexyl. Ferner können die Alkenylreste auch mehrere Doppelbindungen enthalten.

Die Ausgangsstoffe der chemischen Umsetzungen sind bekannt oder lassen sich nach literaturbekannten Methoden leicht herstellen.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, daß man
a) eine Aminocarbonsäure der Formel III, 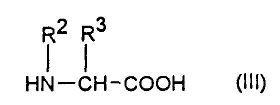 worin R² und R³ wie in Formel I definiert sind, mit einem Sulfonsäurederivat der Formel IV, 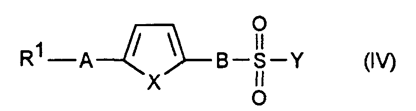 worin R¹, A und B wie in Formel I definiert sind und Y ein Halogenatom, Imidazolyl oder -OR⁸ bedeutet, worin R⁸ Wasserstoffatom, (C₁-C₆)-Alkyl, Phenyl oder Benzyl, gegebenenfalls substituiert darstellt,
   in Gegenwart einer Base oder gegebenenfalls eines wasserentziehenden Mittels zu einer Verbindung der Formel I umsetzt, oder
b) einen Aminocarbonsäureester der Formel V, 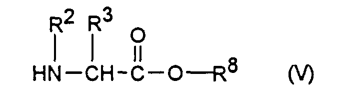 worin R², R³ und R⁸ die obengenannte Bedeutung haben, mit einem Sulfonsäurederivat der Formel IV unter den obengenannten Bedingungen zu einer Verbindung der Formel VI 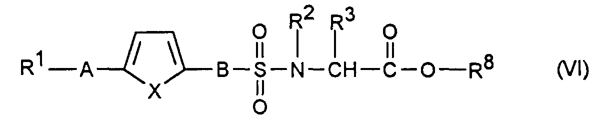 umsetzt, und die Verbindung der Formel VI unter Abspaltung des Restes R⁸, bevorzugt in Gegenwart einer Base oder Säure in eine Verbindung der Formel I umwandelt, oder
c) die Verbindung der Formel VII, 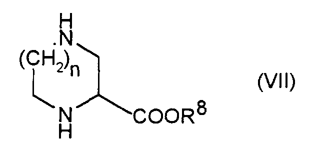 wobei n die ganze Zahl Null, 1 oder 2 darstellt, mit Hilfe einer Schutzgruppe E zu einer Verbindung der Formel VIII umsetzt, 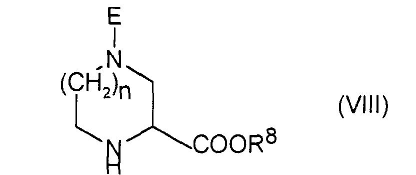 die Verbindung der Formel VIII mit einem Sulfonsäurederivat der Formel IV unter den obengenannten Bedingungen in eine Verbindung der Formel IX überführt 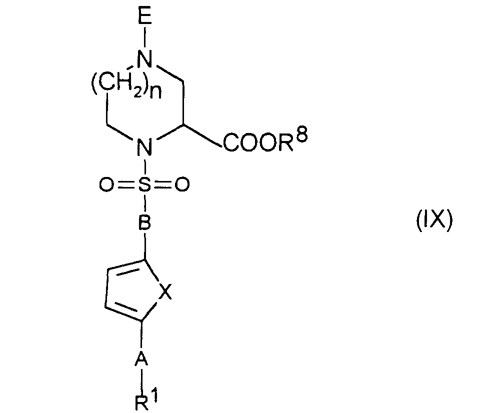 und anschließend die Verbindung der Formel IX unter Abspaltung der Schutzgruppe E und des Restes R⁸ mit Hilfe geeigneter Spaltreagenzien in die Verbindung der Formel I überführt, oder
d) eine nach den Verfahren a), b) oder c) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
e) die nach den Verfahren a), b), c) oder d) hergestellte Verbindung der Formel I entweder in freier Form isoliert oder im Falle des Vorliegens von sauren oder basischen Gruppen in physiologisch verträgliche Salze umwandelt.

Als geeignete Schutzgruppe E werden dafür vorzugsweise die in der Peptidchemie gebräuchlichen N-Schutzgruppen verwendet, beispielsweise Schutzgruppen vom Urethan-Typ, Benzyloxycarbonyl (Z), t-Butyloxycarbonyl (Boc), 9-Fluorenyloxycarbonyl (Fmoc), Allyloxycarbonyl (Aloc) oder vom Säureamid-Typ insbesondere Formyl, Acetyl oder Trifluoracetyl sowie vom Alkyl-Typ beispielsweise Benzyl.

Als Verbindungen der Formel III, bei denen R² ein Wasserstoffatom und R³ der charakteristische Rest einer natürlichen α-Aminosäure bedeutet, werden vorzugsweise Glycin, Alanin, Valin, Leucin, Isoleucin, Phenylalanin, Tyrosin, Tryptophan, Serin, Threonin, Cystein, Methionin, Asparagin, Glutamin, Lysin, Histidin, Arginin, Glutaminsäure und Asparaginsäure eingesetzt. Bei natürlichen aber auch nichtnatürlichen α-Aminosäuren, die eine funktionelle Gruppe wie Amino, Hydroxy, Carboxy, Mercapto, Guanidyl, Imidazolyl oder Indolyl in der Seitenkette R³ haben, kann diese Gruppe auch geschützt sein.

Für den Fall eines Imidazols-Restes in R³ dient beispielsweise das für die Sulfonamidbildung eingesetzte Sulfonsäurederivat der Formel IV als Schutzgruppe des Imidazol-Stickstoffs, die sich insbesondere in Gegenwart von Basen wie Natronlauge wieder abspalten läßt.

Zur Darstellung von Verbindungen der Formel I, in denen R² und R³ zusammen einen Ring der Teilstruktur II bilden, werden als Ausgangsstoffe der Formel III beispielsweise Prolin, 3- oder 4-Hydroxyprolin, Piperidin-2-carbonsäure, Piperazin-2-carbonsäure und Hexahydropyridazin-3-carbonsäure benutzt, wobei insbesondere der Stickstoff in 4-Stellung der Piperazin-2-carbonsäure durch eine Schutzgruppe Z, beispielsweise Benzyloxycarbonyl- oder tert.-Butyloxycarbonyl wie in der Verfahrensvariante c) beschrieben oder durch einen Rest R⁷ substituiert sein kann.

Als Ausgangsprodukte zur Darstellung der Sulfonsäurederivate der Formel IV dienen bevorzugt Sulfonsäuren oder deren Salze der Formel X, beispielsweise 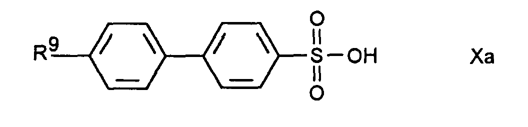 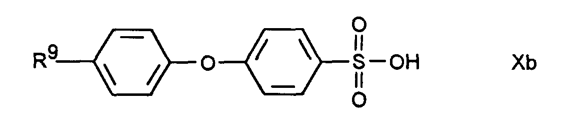 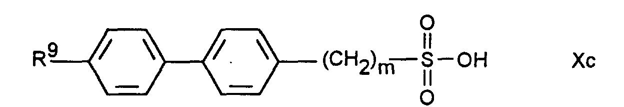 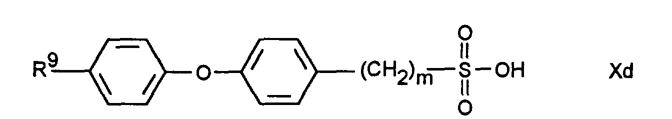 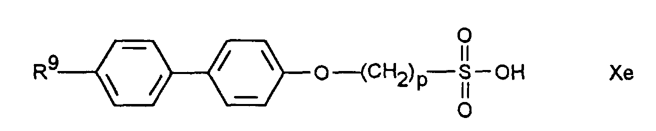 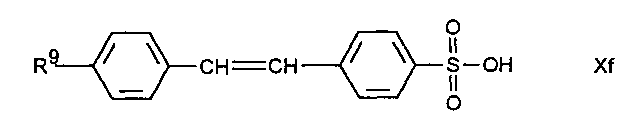 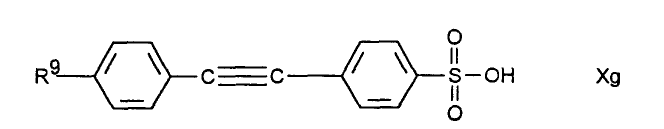 wobei R⁹ ein unter 2.1. bis 2.14. beschriebener Rest bedeutet.

Zur Herstellung der Arylsulfonsäuren der Formeln Xa und b bedient man sich vorzugsweise der im Houben-Weyl "Methoden der Organischen Chemie" Band 9, S. 450-546 beschriebenen Sulfonierungsverfahren mit konzentrierter Schwefelsäure ggf. in Gegenwart eines Katalysators, Schwefeltrioxids und seinen Additionsverbindungen oder Halogensulfonsäuren, wie Chlorsulfonsäure. Besonders im Falle der Diphenylether der Formel Xb hat sich die Verwendung von konzentrierte Schwefelsäure und Essigsäureanhydrid als Lösemittel (vergl. C.M. Suter, J. Am. Chem. Soc. 53 (1931) 1114), oder die Umsetzung mit überschüssiger Chlorsulfonsäure (J.P. Bassin, R. Cremlyn und F. Swinbourne; Phosphorus, Sulfur and Silicon 72 (1992) 157) bewährt. Sulfonsäuren gemäß der Formel Xc, Xd, oder Xe lassen sich in an sich bekannter Weise herstellen, in dem man das entsprechende Arylalkylhalogenid mit Sulfiten wie Natriumsulfit oder Ammoniumsulfit in wäßriger oder wäßrig/alkoholischer Lösung umsetzt, wobei die Umsetzung in Gegenwart von Tetraorganoammoniumsalzen wie Tetrabutylammoniumchlorid beschleunigt werden kann.

Als Sulfonsäurederivate gemäß Formel IV finden insbesondere die Sulfonsäurechloride Verwendung. Zu ihrer Herstellung werden die entsprechenden Sulfonsäuren, auch in Form ihrer Salze wie Natrium-, Ammonium- oder Pyridiniumsalze in bekannter Weise mit Phosphorpentachlorid oder Thionylchlorid ohne oder in Gegenwart eines Lösemittels wie Phosphoroxytrichlorid oder eines inerten Lösemittels wie Methylenchlorid, Cyclohexan oder Chloroform im allgemeinen bei Reaktionstemperaturen von 20°C bis zum Siedepunkt des verwendeten Reaktionsmediums umgesetzt.

Die Umsetzung der Sulfonsäurederivate der Formel IV mit den Aminosäuren der Formeln III, V oder VII gemäß Verfahrensvarianten a), b) oder c) verläuft vorteilhaft nach Art der Schotten-Baumann-Reaktion. Als Base eignen sich dafür besonders Alkalihydroxide wie Natriumhydroxid, aber auch Alkaliacetate, -hydrogencarbonate,-carbonate und Amine. Die Umsetzung findet in Wasser oder in einem mit Wasser mischbaren oder nichtmischbaren Lösemittel wie Tetrahydrofuran (THF), Aceton, Dioxan oder Acetonitril statt, wobei die Reaktionstemperatur im allgemeinen von -10°C bis 50°C gehalten wird. Für den Fall, daß die Reaktion im wasserfreien Medium durchgeführt wird, findet vor allem Tetrahydrofuran oder Methylenchlorid, Acetonitril oder Dioxan in Gegenwart einer Base, wie Triethylamin, N-Methylmorpholin, N-Ethyl- oder Diisopropylethylamin Verwendung, eventuell in Gegenwart von N,N-Dimethylaminopyridin als Katalysator.

In einer anderen Variante kann man die Aminocarbonsäuren der Formel III, IV oder VII zuerst mit Hilfe eines Silylierungsmittels wie Bis-trimethylsilyltrifluoracetamid (BSTFA) in ihre silylierte Form überführen und sie dann mit Sulfonsäurederivaten zu Verbindungen der Formel I umsetzen.

Die Herstellung physiologisch verträglicher Salze aus zur Salzbildung befähigten Verbindungen der Formel I, einschließlich deren stereoisomeren Formen, erfolgt in an sich bekannter Weise. Die Carbonsäuren bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder Triethanolamin oder auch basischen Aminosäuren, etwa Lysin, Ornithin oder Arginin, stabile Alkali-, Erdalkali- oder gegebenenfalls substituierte Ammoniumsalze. Sofern die Verbindungen der Formeln I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-,
Phosphor-, Methansulfon-, Benzolsulfon-, p-Toluolsulfon-, 4-Brombenzol-sulfon-, Cyclohexylamidosulfon-, Trifluormethylsulfon-, Essig-, Oxal-, Wein-, Bernstein- oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur Prophylaxe und Therapie all solcher Erkrankungen, an deren Verlauf eine verstärkte Aktivität von Matrix-abbauenden Metalloproteinasen beteiligt ist. Dazu gehören degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Ferner gehören dazu auch Erkrankungen des Bindegewebes wie Kollagenosen, Periodontalerkrankungen, Wundheilungs-störungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels. Ferner eignen sich die Verbindungen der Formel I zur Behandlung der Ulceration, Atherosklerose und Stenosen. Weiterhin eignen sich die Verbindungen der Formel I zur Behandlung von Entzündungen, Krebserkrankungen, Tumormetastasenbildung, Kachexie, Anorexie und septischem Schock.

Die erfindungsgemäßen Arzneimittel werden im allgemeinen oral oder parenteral verabreicht. Die rektale oder transdermale Applikation ist auch möglich.

Die Erfindung betrifft auch ein Verfahren zur Herstellung eines Arzneimittels, das dadurch gekennzeichnet, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien Magnesium-carbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind - je nach Wirksamkeit der Verbindungen gemäß Formel I, Tagesdosen von etwa 20 mg bis 1000 mg Wirkstoff, bevorzugt etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

¹H-NMR-Spektren sind an einem 200-MHz-Gerät der Firma Varian aufgenommen worden, in der Regel mit Tetramethylsilan (TMS) als internem Standard und bei Raumtemperatur (RT). Die verwendeten Lösemittel sind jeweils angegeben. Endprodukte werden in der Regel durch massenspektroskopische Methoden (FAB-, ESI-MS) bestimmt. Temperaturangaben in Grad Celsius, RT bedeutet Raumtemperatur (22-26°C). Verwendete Abkürzungen sind entweder erläutert oder entsprechen den üblichen Konventionen.

### Beispiel 1: N-(Phenoxybenzolsulfonyl)-homoserin (hergestellt nach Verfahrensvariante a)

10 g (54,9 mmol) D,L-Homoserin-lacton werden unter Eiskühlung mit 50 ml 1N NaOH und 50 ml Tetrahydrofuran (THF)versetzt. Bei 5°C wird unter Rühren 16,0 g (59,5 mmol) Phenyloxybenzolsulfonsäurechlorid in 50 ml THF zugetropft, wobei nach der Hälfte der Zugabe das Reaktionsgemisch mit 7,1 g (54,9 mmol) Diisopropylethylamin versetzt wird. Nach Rühren über Nacht wird mit 2N HCI auf pH = 5,5 eingestellt und mehrmals mit Eisessig extrahiert. Die vereinigten organischen Phasen werden, nach Trocknen über Natriumsulfat, filtriert und unter verminderten Druck eingedampft. Die Umkristallisation aus Eisessig/Petrolether ergibt die obengenannte Verbindung.

Ausbeute: 18,3 g (73 % der Theorie) Schmelzpunkt: 134°C

¹H-NMR (DMSO- d6): 1,6 - 1,85 (m, 2H); 3,2 - 3,45 (m, 3H); 3,75 - 3,95 (m, 1H); 7,0-8,1 (m, 9H)

### Beispiel 2: (2R)-1-(4-Chlorbiphenylsulfonyl)-4-cis-hydroxyprolin (hergestellt nach Verfahrensvariante a)

2 g (15,2 mmol) D-cis-Hydroxyprolin werden in trockenem Acetonitril gelöst und zusammen mit 12,1 ml (46,7 mmol, 3,1 Äquivalente) BSTFA (Bis-trimethylsilyltrifluoracetamid) für 2 Stunden unter Rückfluß erhitzt. Anschließend wird mit 4,4 g (15,2 mmol) 4-Chlorbiphenylsulfonsäurechlorid in 15 ml Acetonitril versetzt und für weitere 4 Stunden unter Rückfluß belassen. Es bildet sich ein dicker, weißer Niederschlag der O-silylierten- N-sulfonierten Verbindung. Nach dem Abkühlen der Suspension und Vervollständigung der Fällung wird dieser abgetrennt und unter verminderten Druck gut getrocknet. Die Ausbeute der Umsetzung ist quantitativ.

Zur Desilylierung weren 100 mg der O-silylierten Verbindung in 10 ml Methanol (MeOH) aufgenommen und mit 10 ml 1N HCI unter Zusatz von 100 mg KF für 2 Stunden bei Raumtemperatur (RT) gerührt. Absaugen des Niederschlags und trocknen unter verminderten Druck ergibt das obengenannte Produkt.

Ausbeute: 61 mg (84 % der Theorie)
¹H-NMR (DMSO- d6): 1,8 - 2,2 (m, 2H); 3,15 (m, 1H); 3,3 (dd, 2H); 4,0 (m, 1H); 4,3 (dd, 1H); 7,6; 7,8 (2d, 4H); 7,9 (s, 4H)

### Beispiel 29: (R)-N-(4-Chlorbiphenylsulfonyl)-tryptophan (hergestellt nach Verfahrensvariante b)

### 29a) (R)-N-(4-Chlorbiphenylsulfonyl)-tryptophan-methylester

5,1 g (20 mmol) D-Tryptophan-methylester-Hydrochlorid wird in 50 ml trockenem Acetonitril suspendiert, mit 2,0 g (20 mmol) Triethylamin versetzt und bei RT gerührt. Nach Zugabe von 6,2 ml (24 mmol) BSTFA wird 2 Stunden bei 80°C gerührt, dann werden 5,75 g (20 mmol) 4-Chlorbiphenylsulfonsäurechlorid in 50 ml Acetonitril und weitere
2,0 g TEA zugetropft und 2 Stunden auf 80°C gehalten. Nach Abkühlen auf RT wird unter Rühren zur Reaktionsmischung 100 ml 1N-HCl gegeben, wobei ein kristalliner Niederschlag ausfällt. Umkristallisation aus Methanol/Wasser ergibt den oben genannten Methylester.
Ausbeute: 6,8 g (92 % der Theorie) Schmelzpunkt: 189°C

### 29 b) (R)-N-(4-Chlorbiphenylsulfonyl)-tryptophan

2,34 g (5 mmol) des obigen Methylesters werden in 30 ml Methanol gelöst und nach Zugabe von 10 ml 1N NaOH 6 Stunden, bei 40°C gerührt. Einstellen der Lösung mit 1N HCI auf pH = 6 ergibt die obengenannte Carbonsäure in kristalliner Form.
Ausbeute: 1,8 g (81% der Theorie) Schmelzpunkt: 138 °C bis 140 °C
¹H-NMR (DMSO- d6): 2,8-2,92 (m,1H), 3,0-3,12 (m, 1H), 3,83-3.97 (m, 1H), 6,85- 7,8 (m, 13 H), 8,3 (d, 1H), 10,75 (s,1H), 12,4 (s, 1H)

Die in der folgenden Tabelle 1 genannten Beispiele sind analog zu den Beispielen 1, 2 und 29 hergestellt worden.

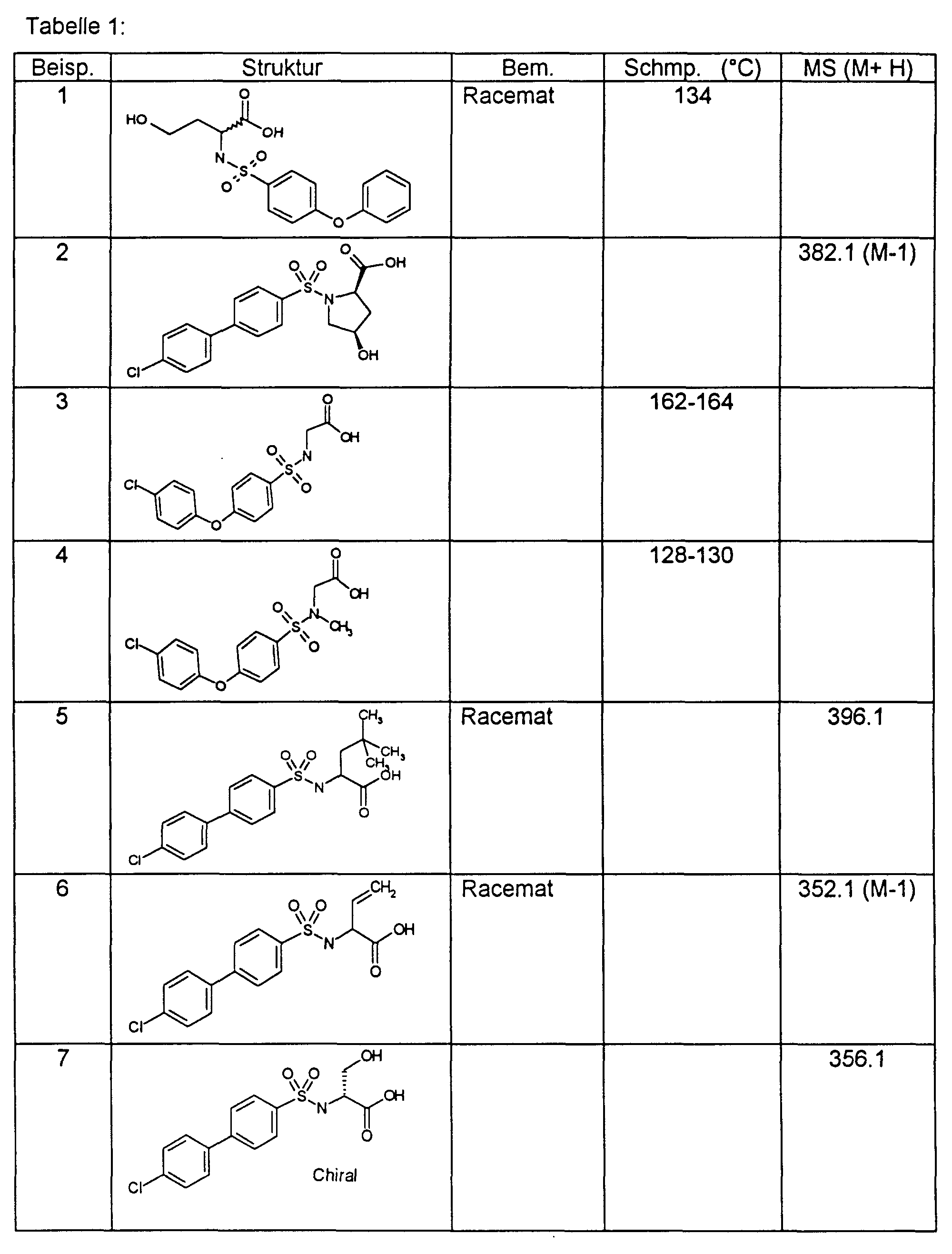

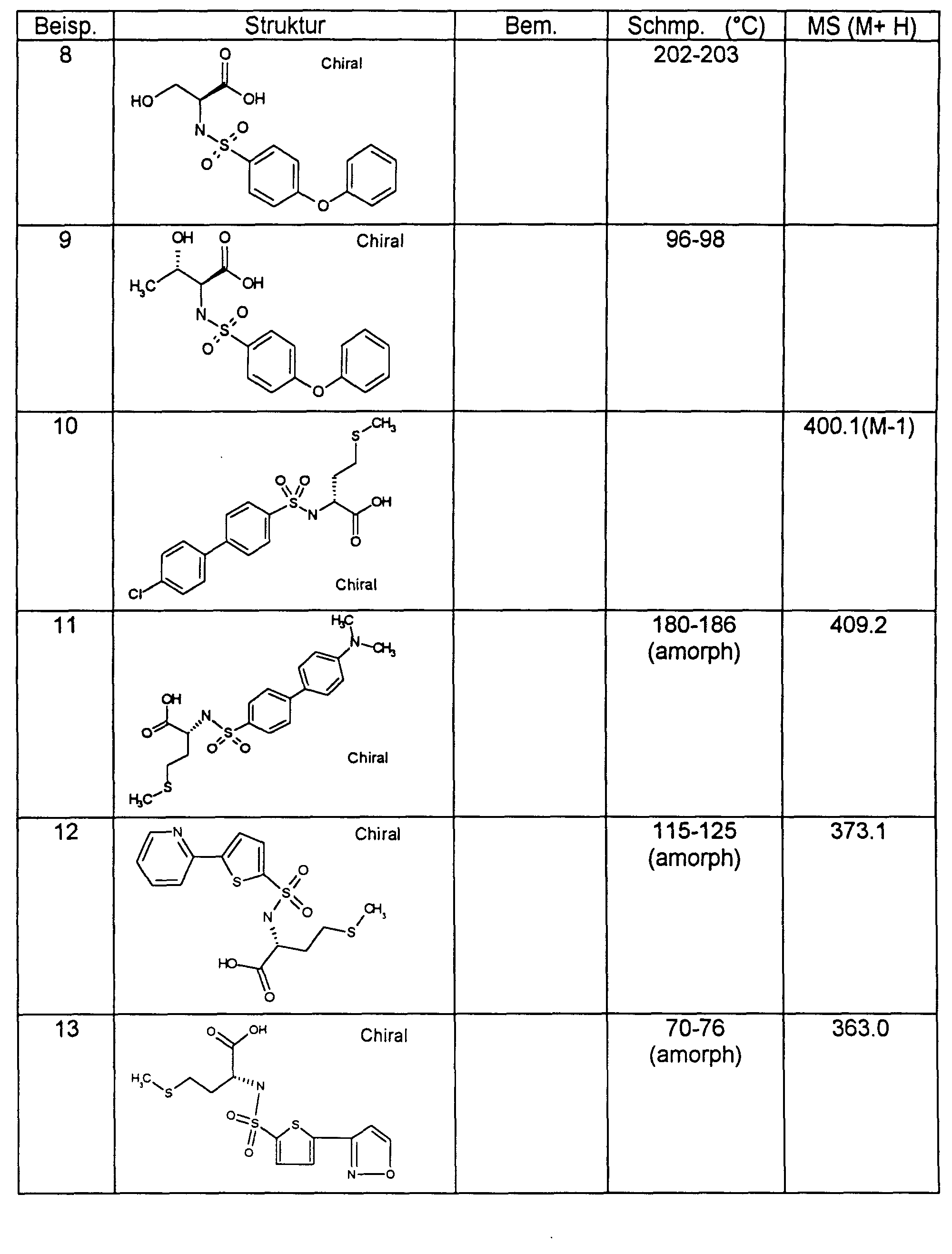

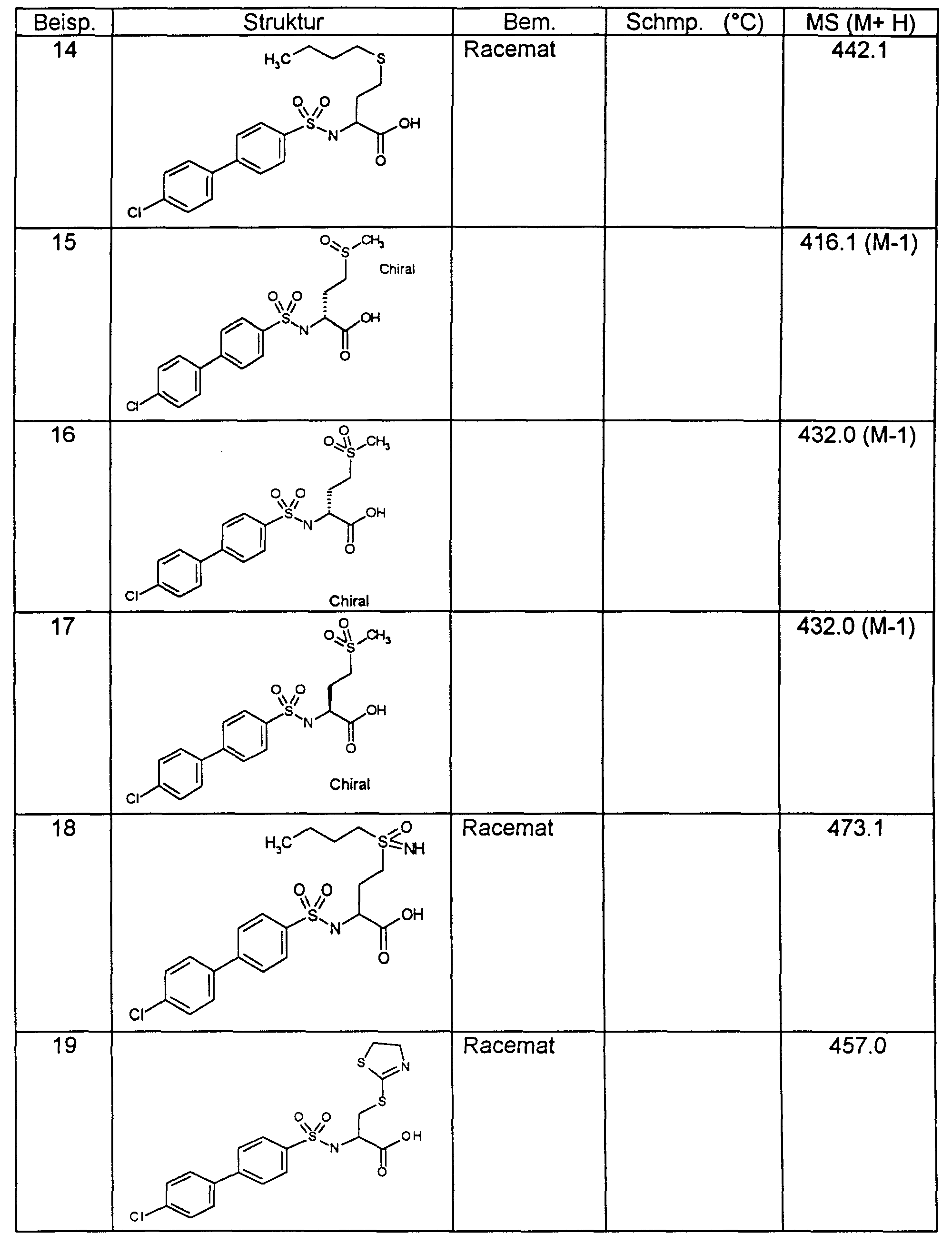

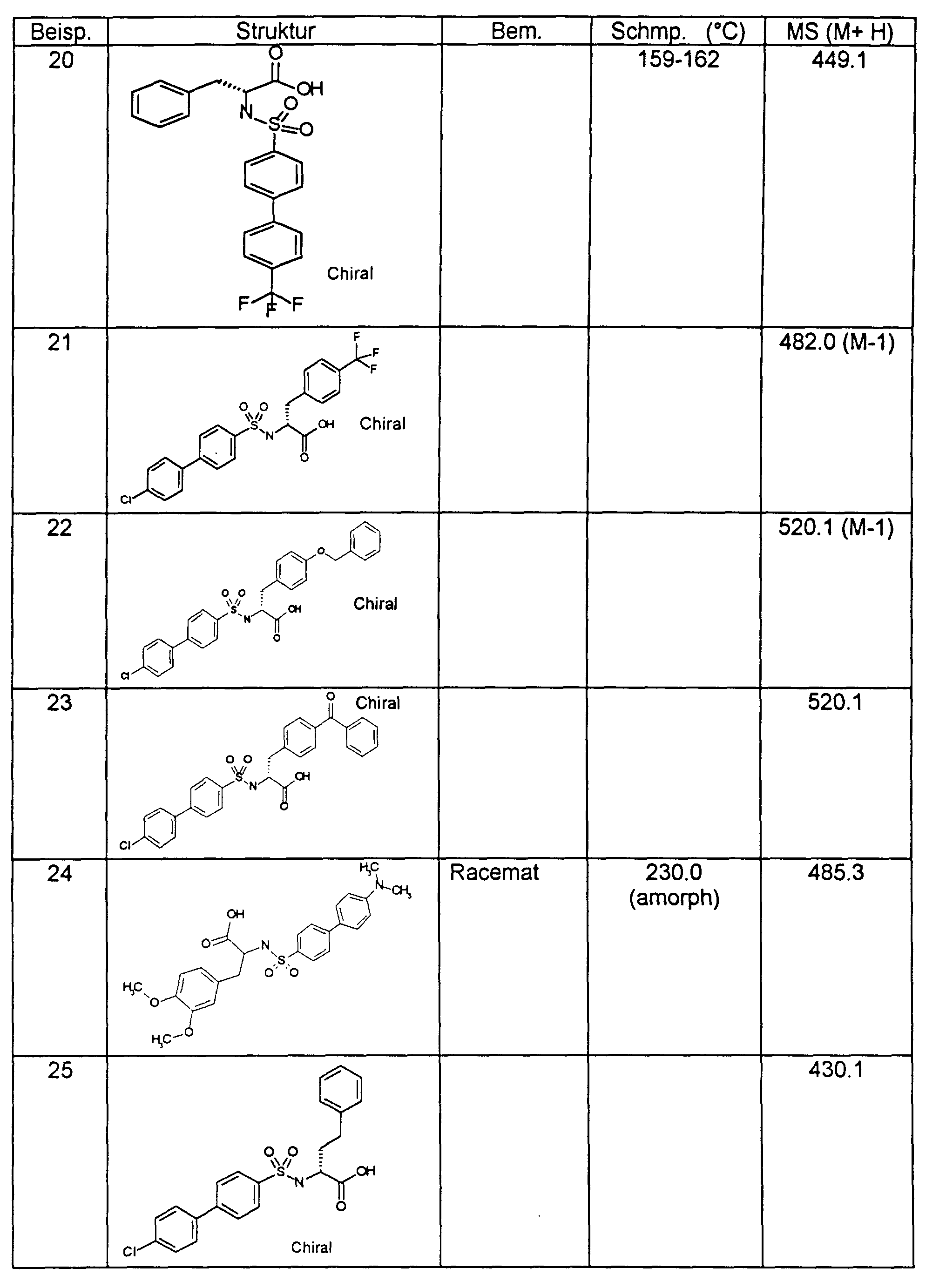

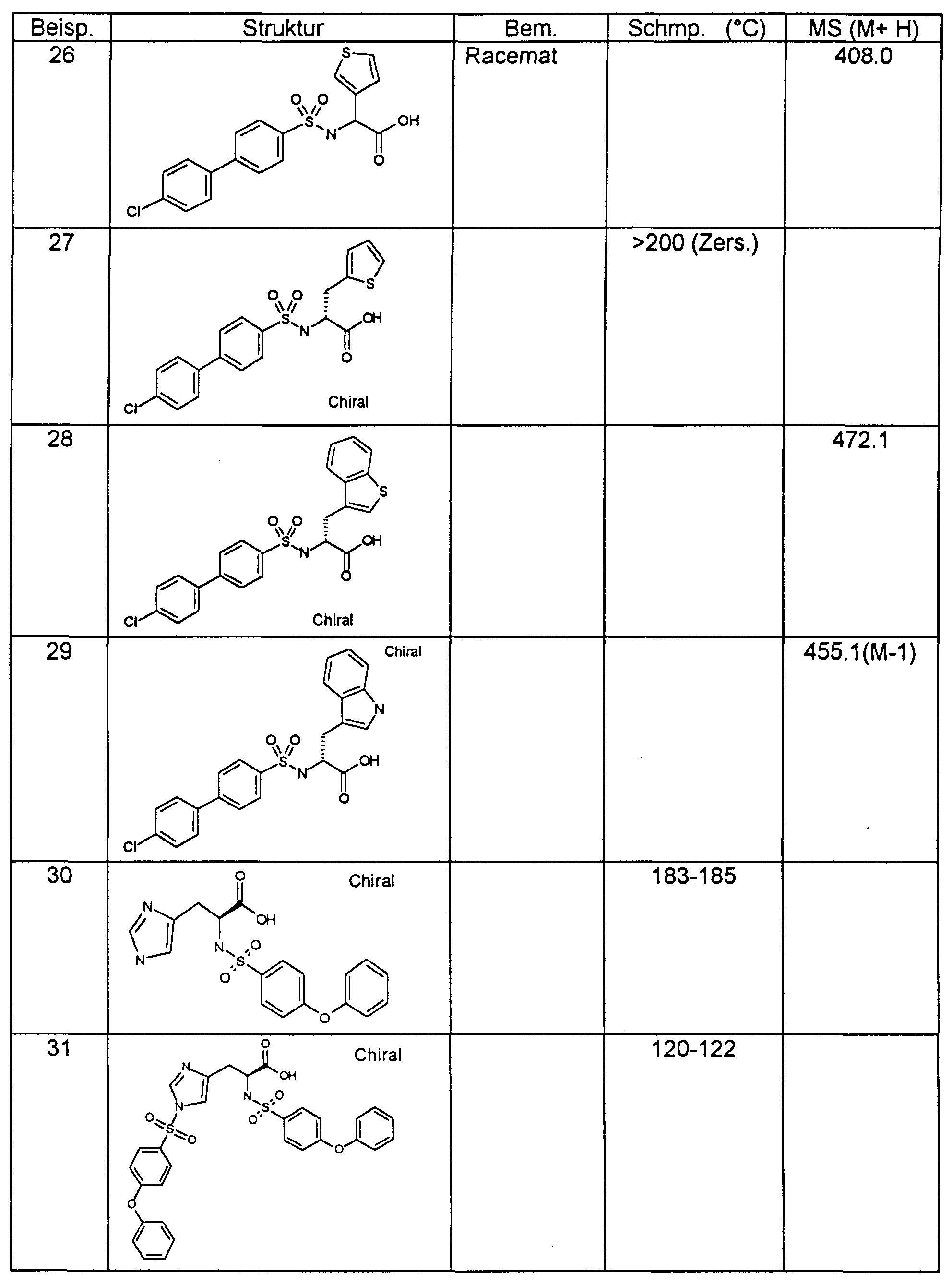

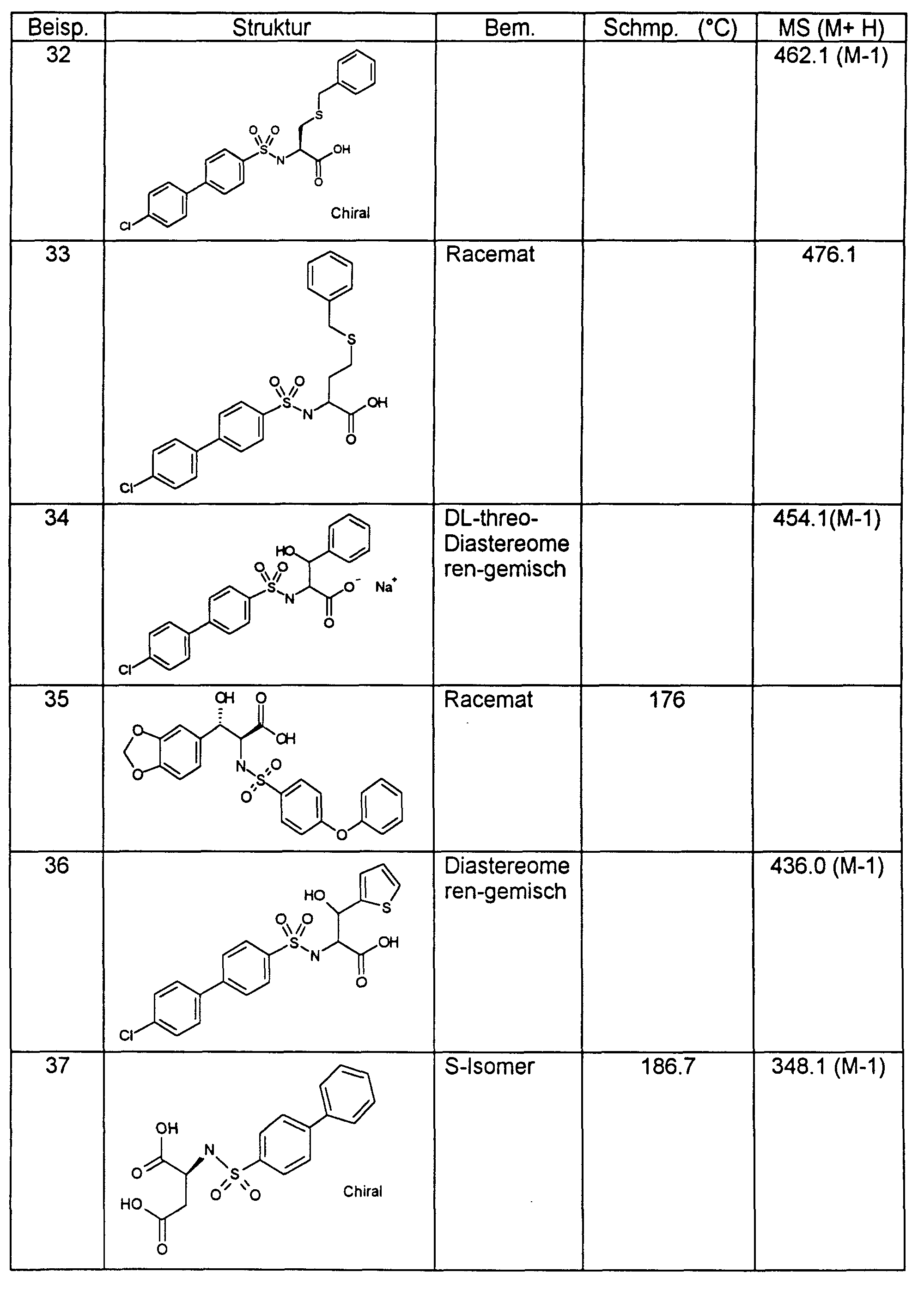

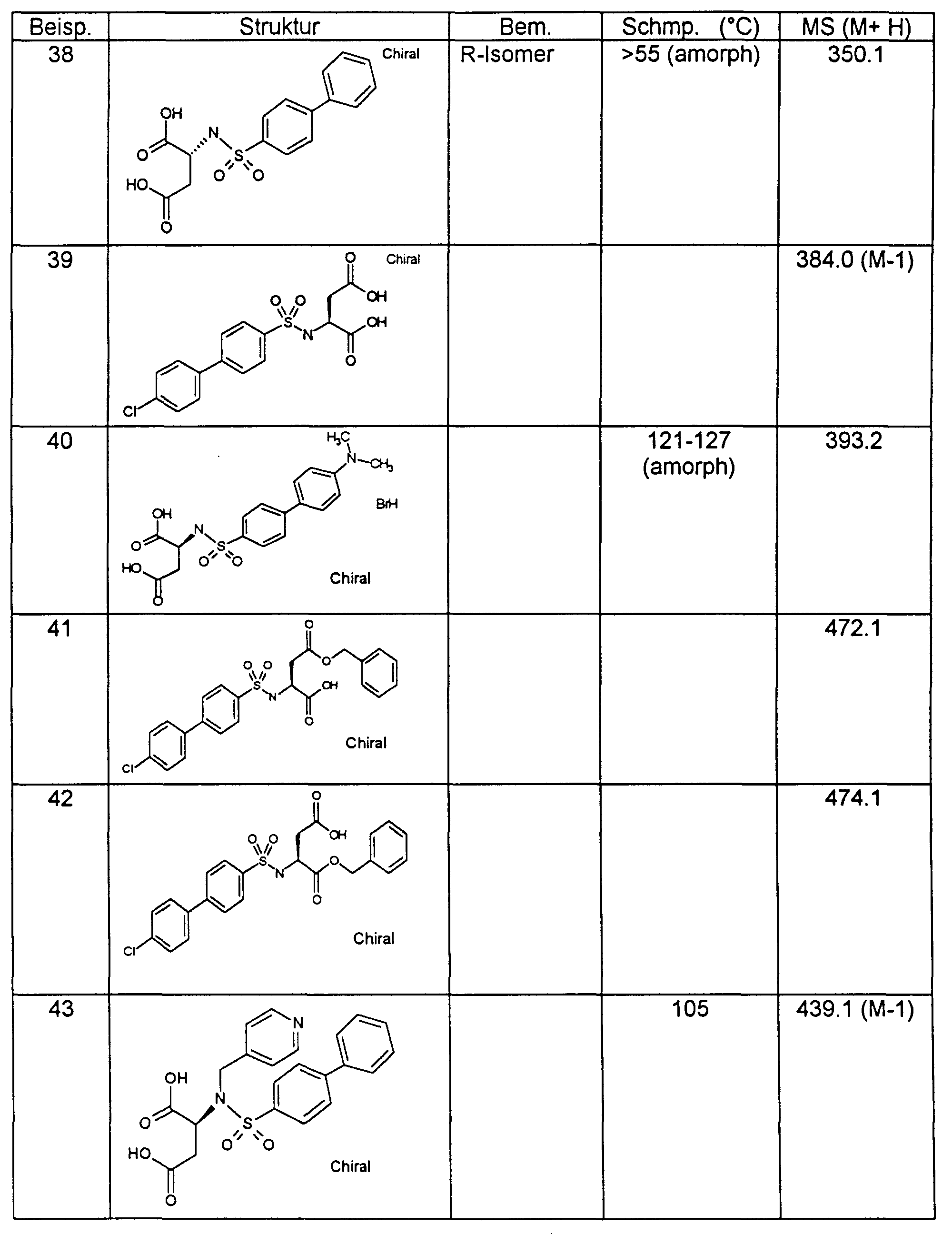

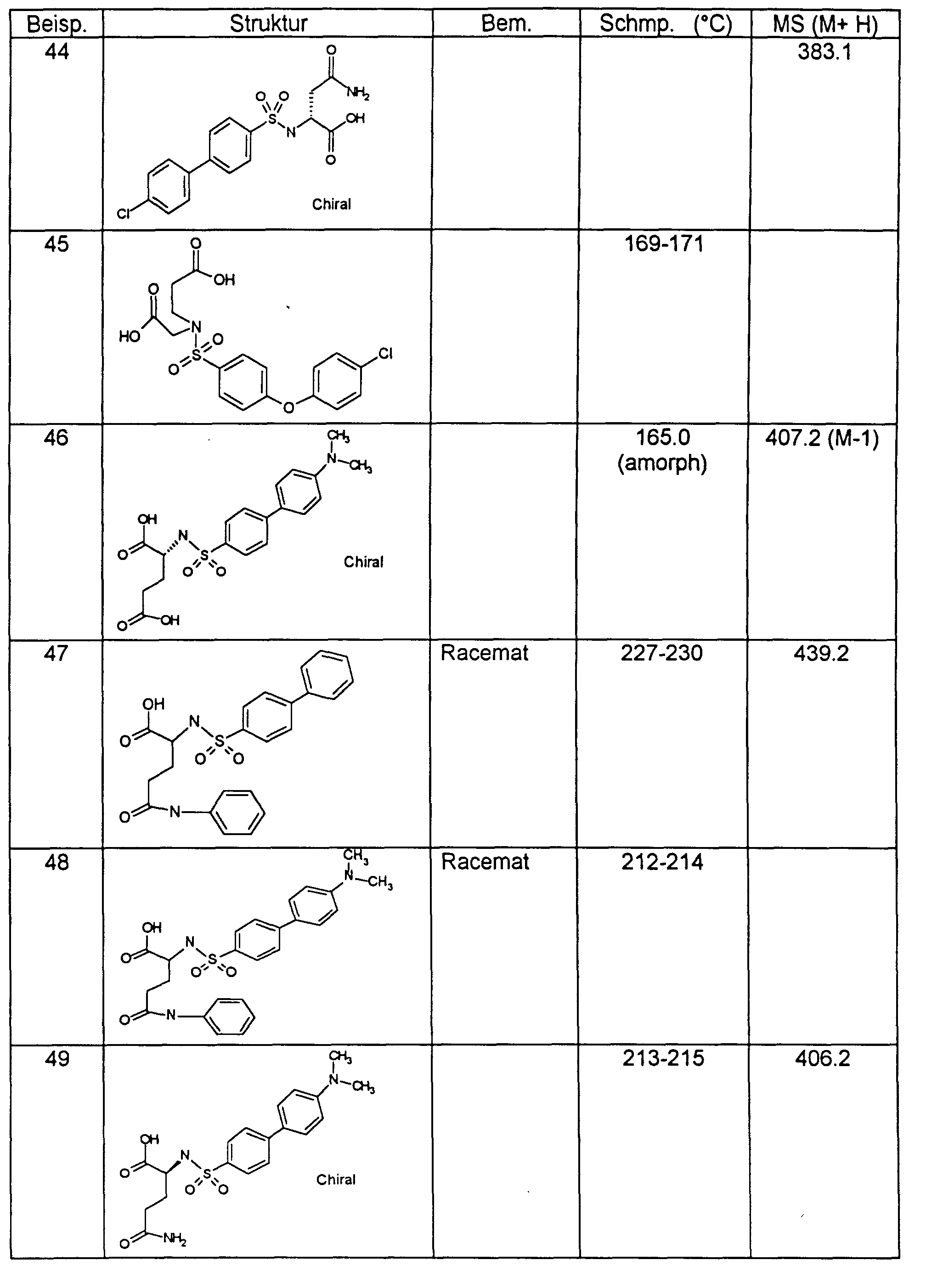

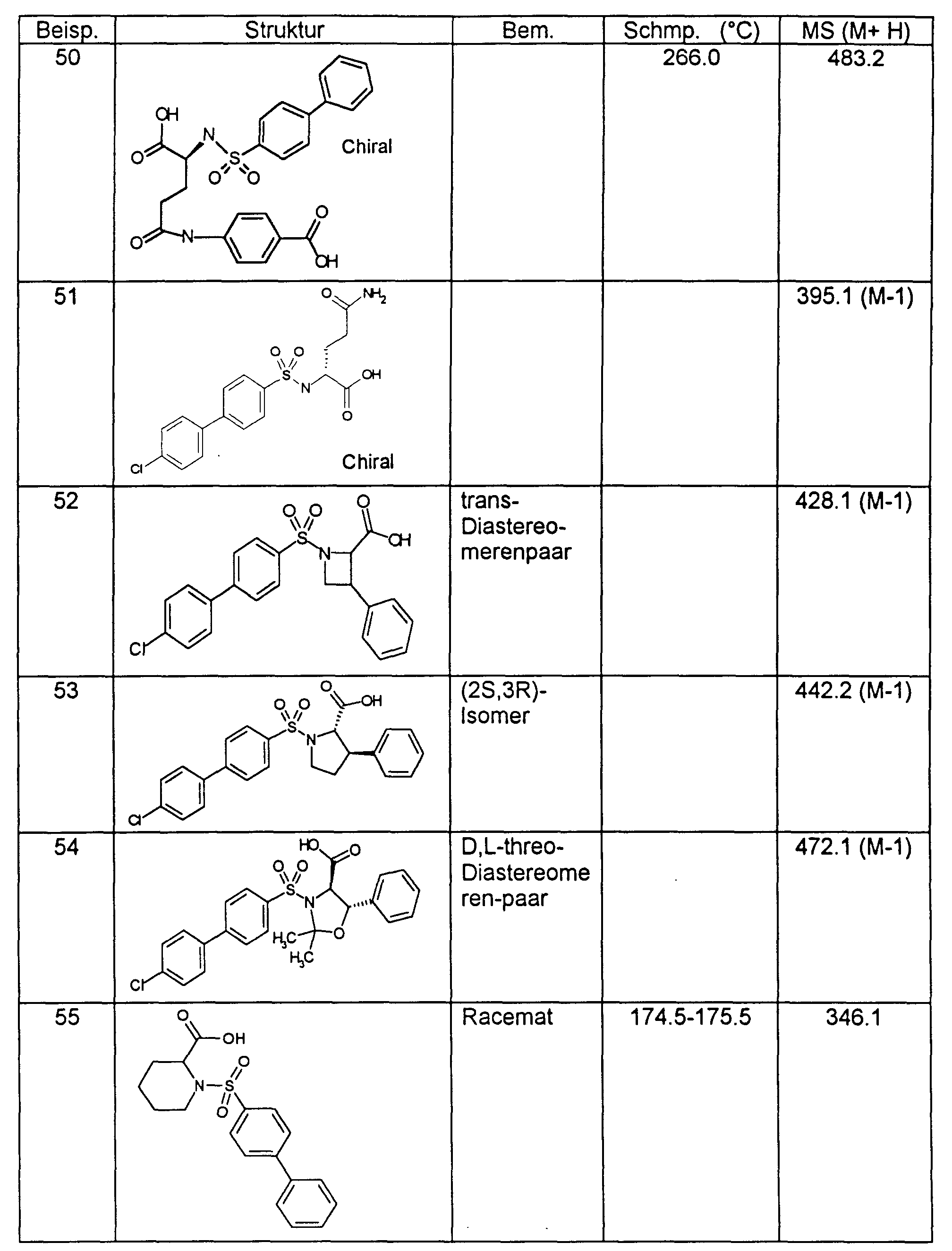

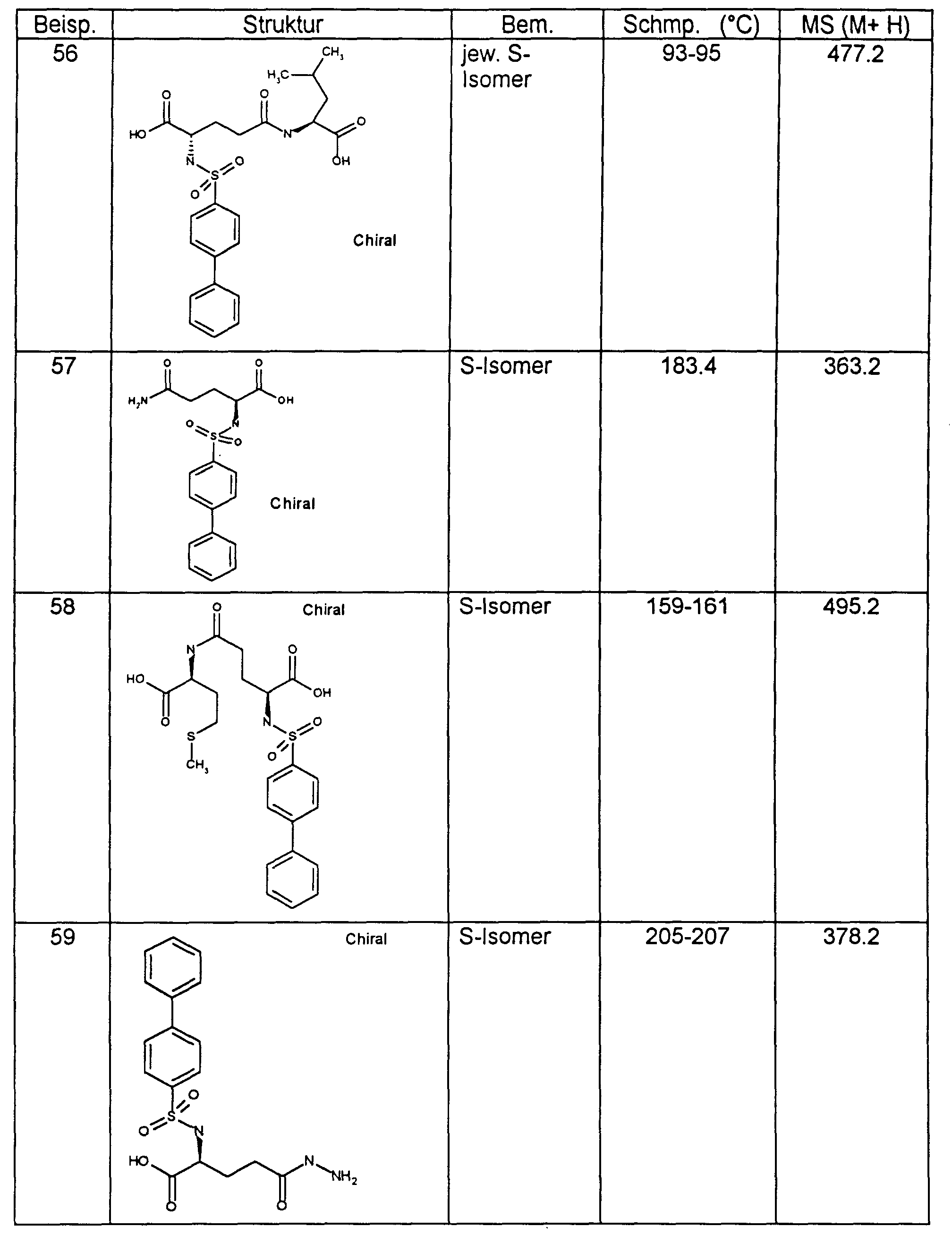

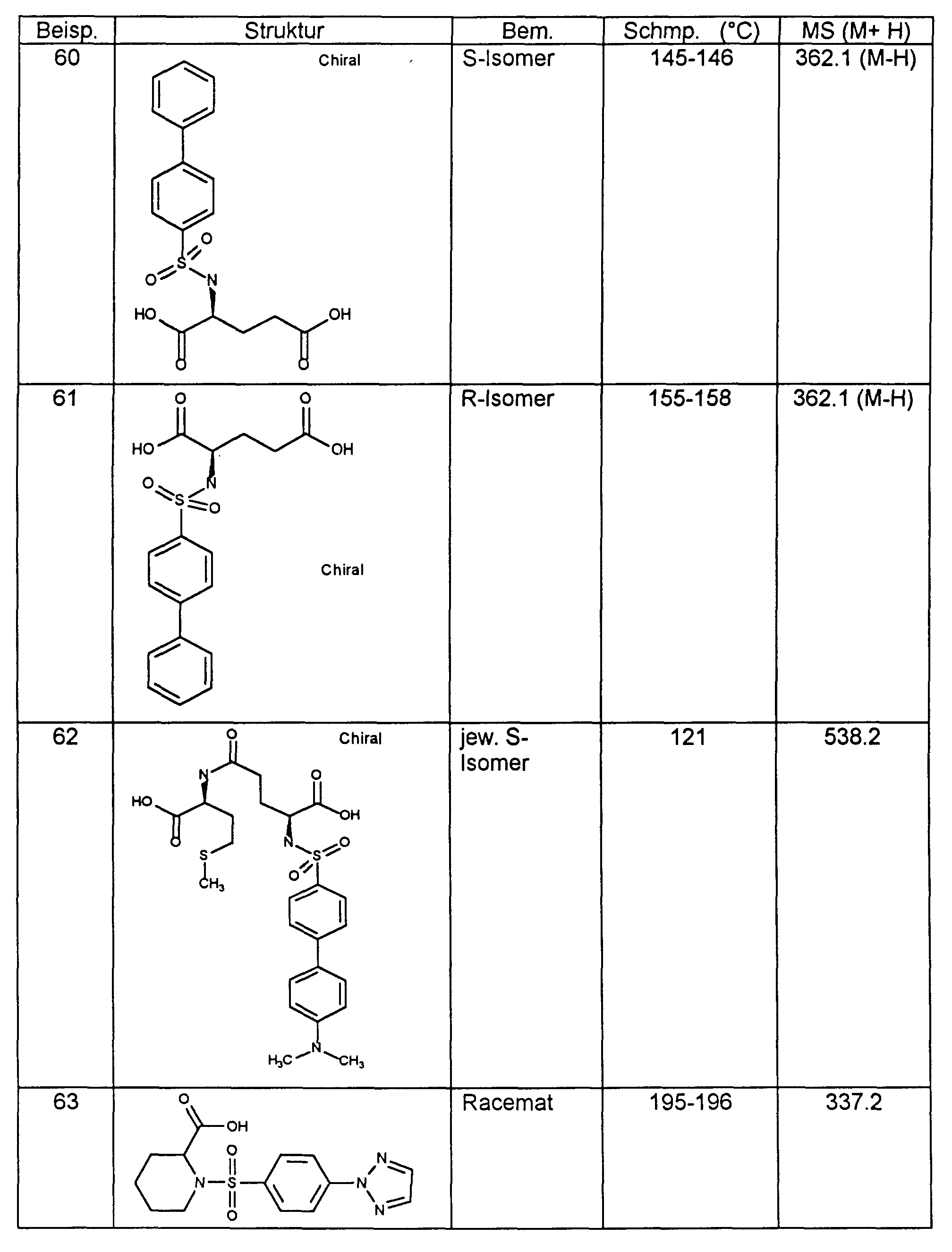

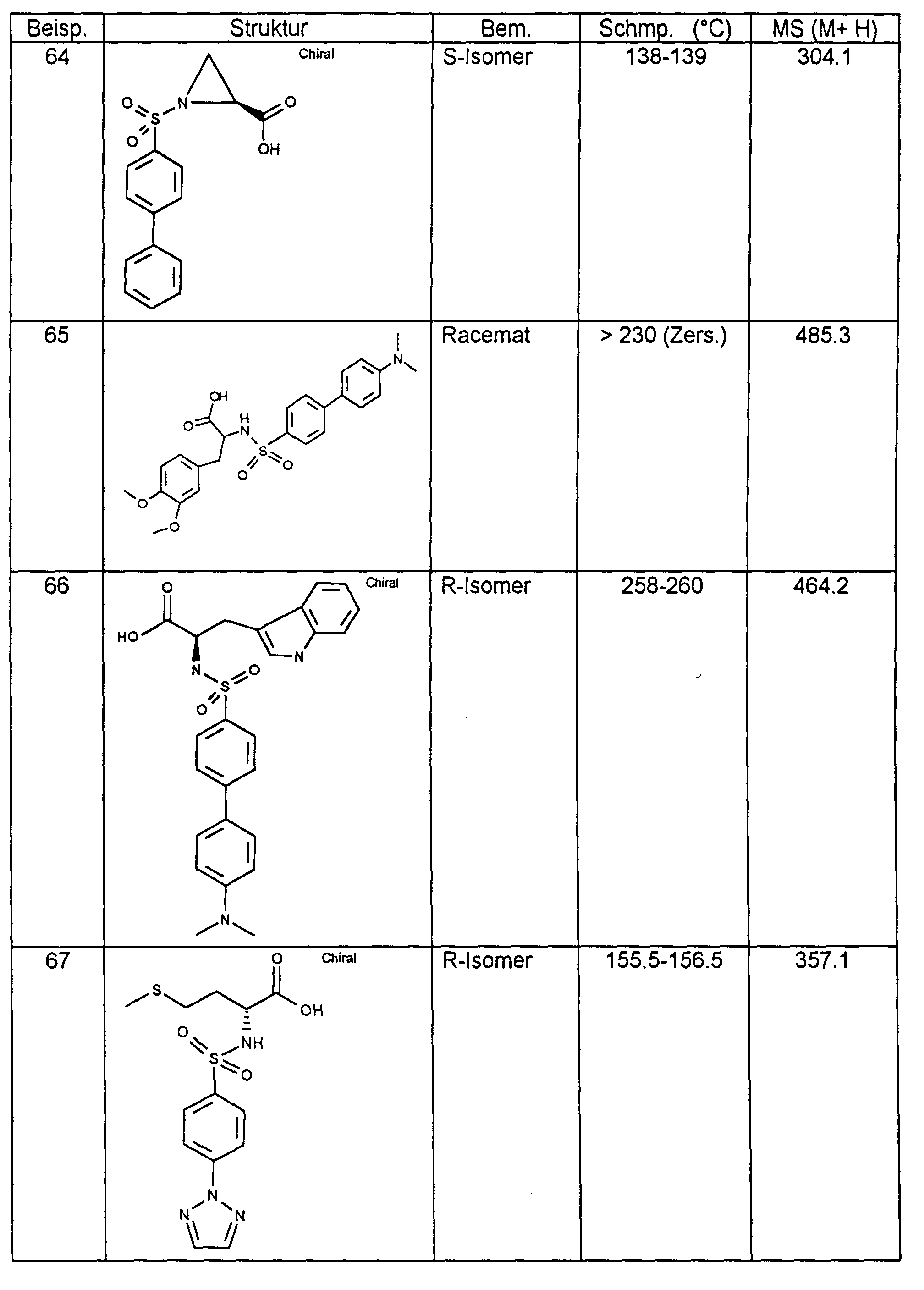

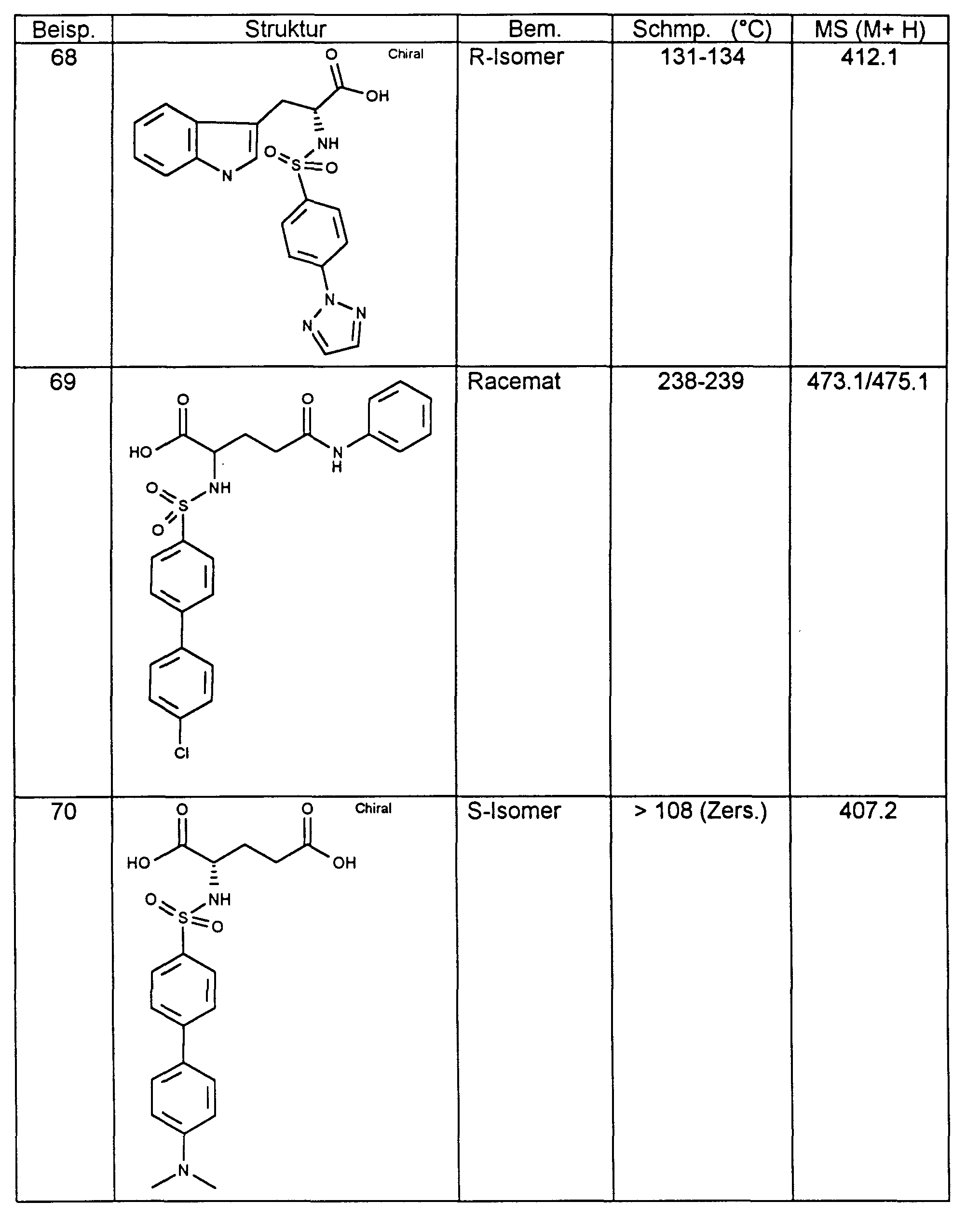

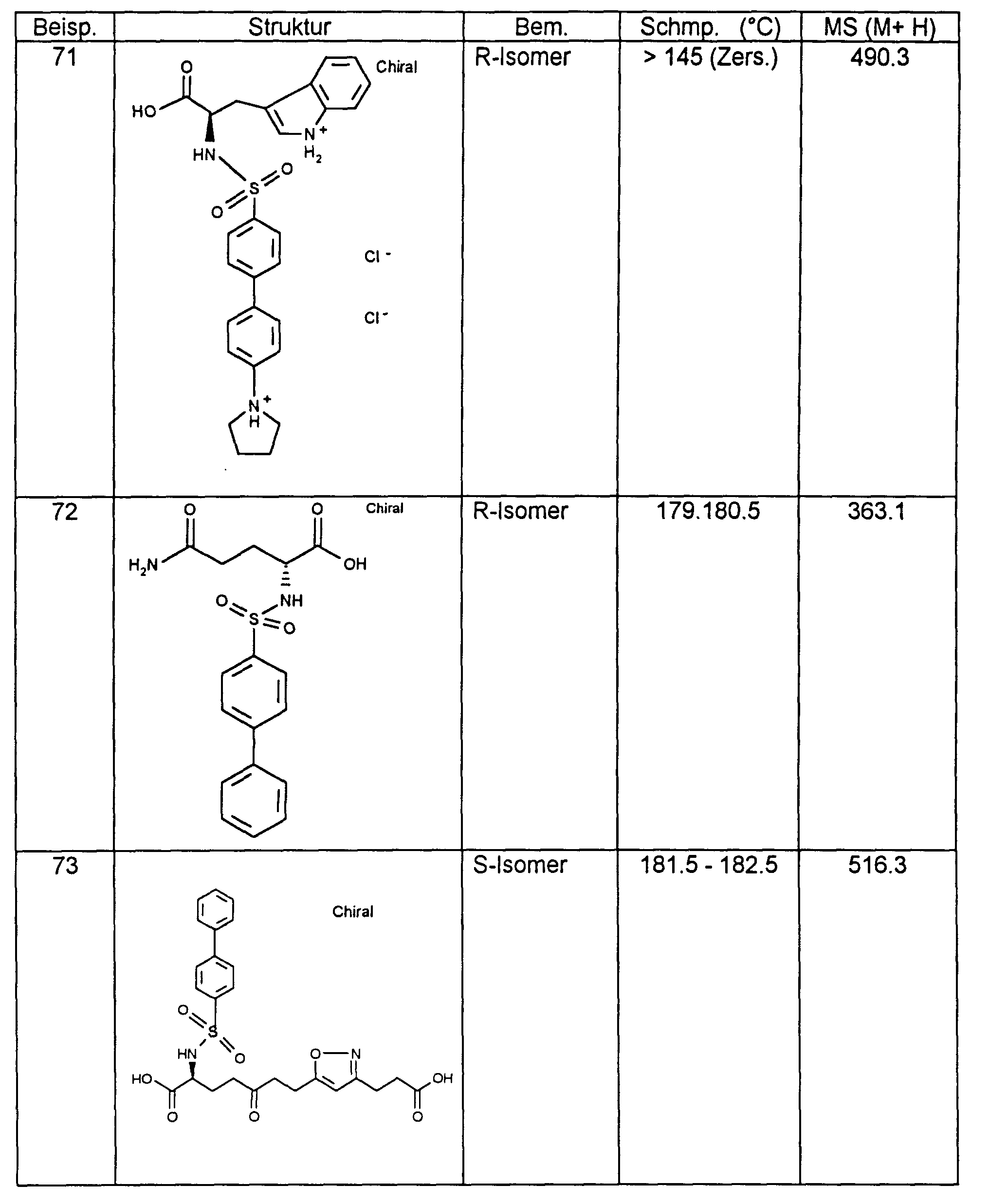

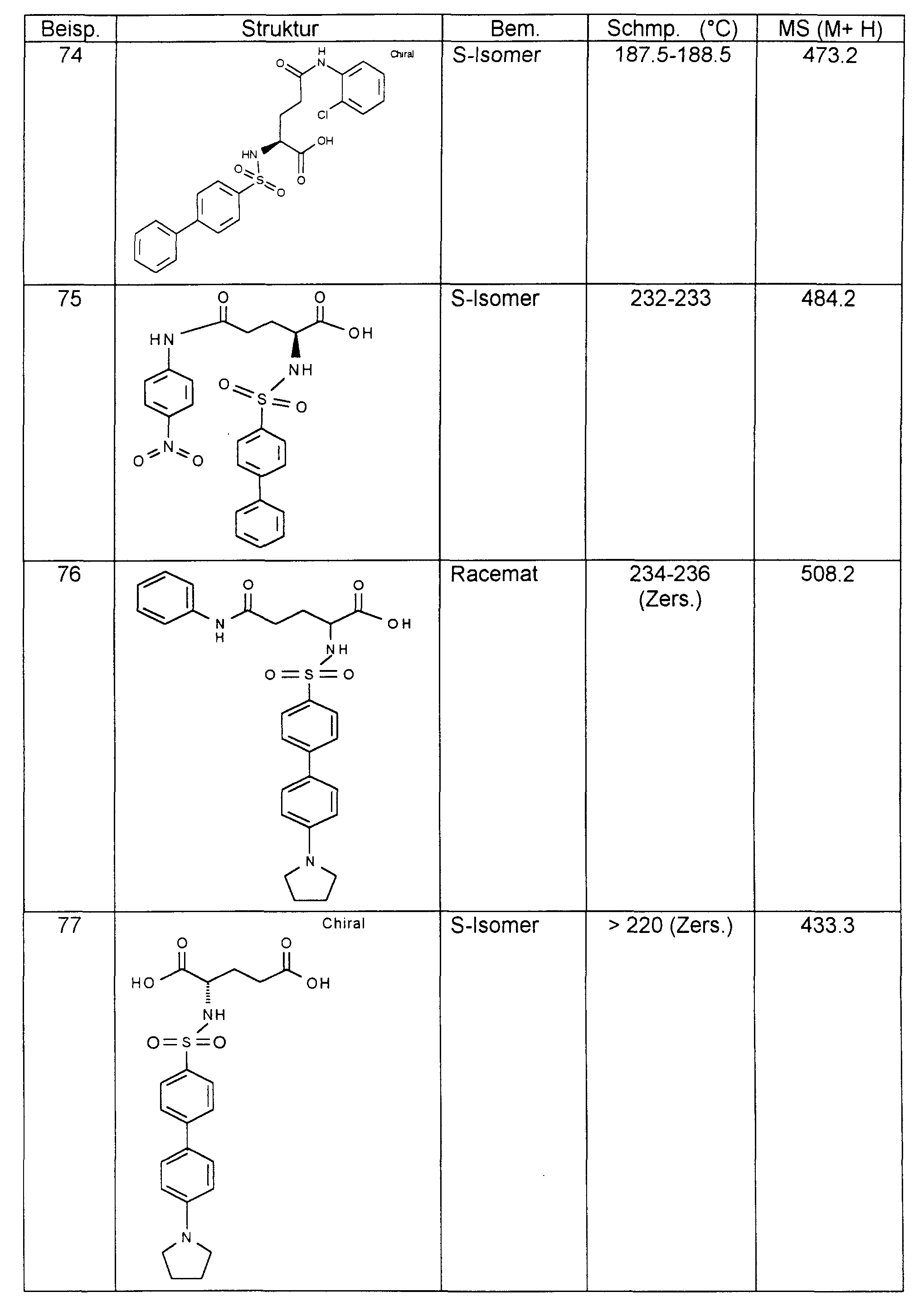

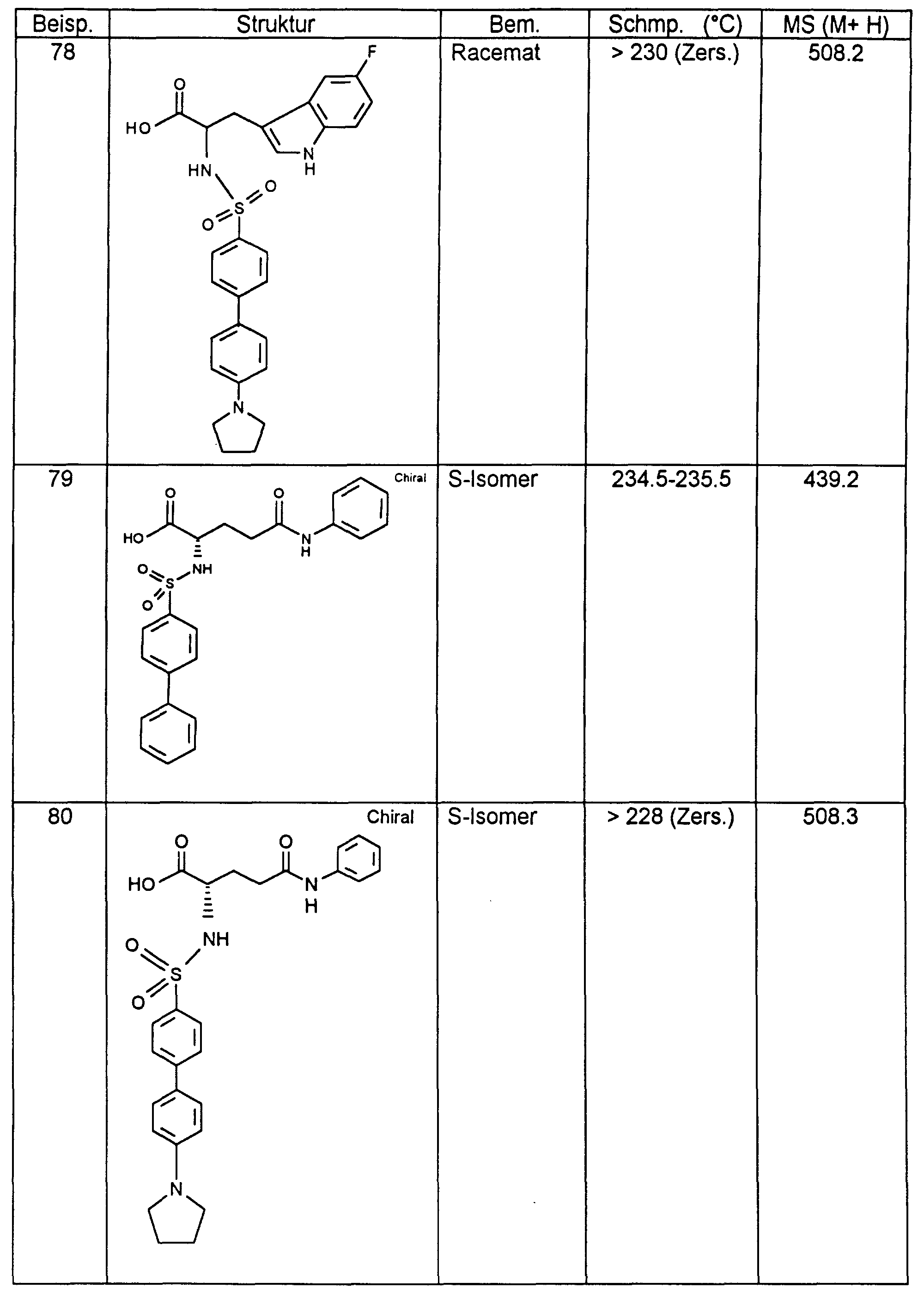

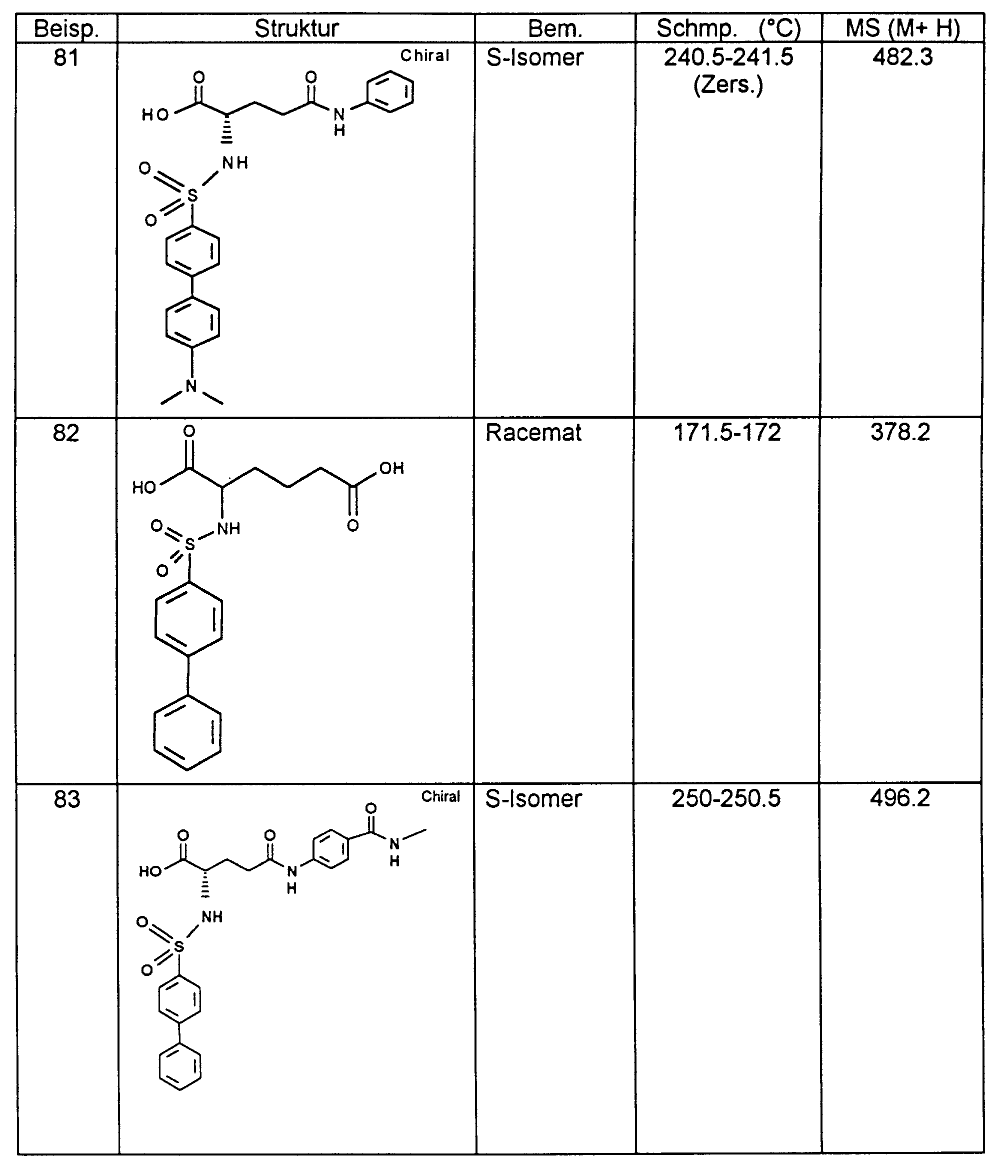

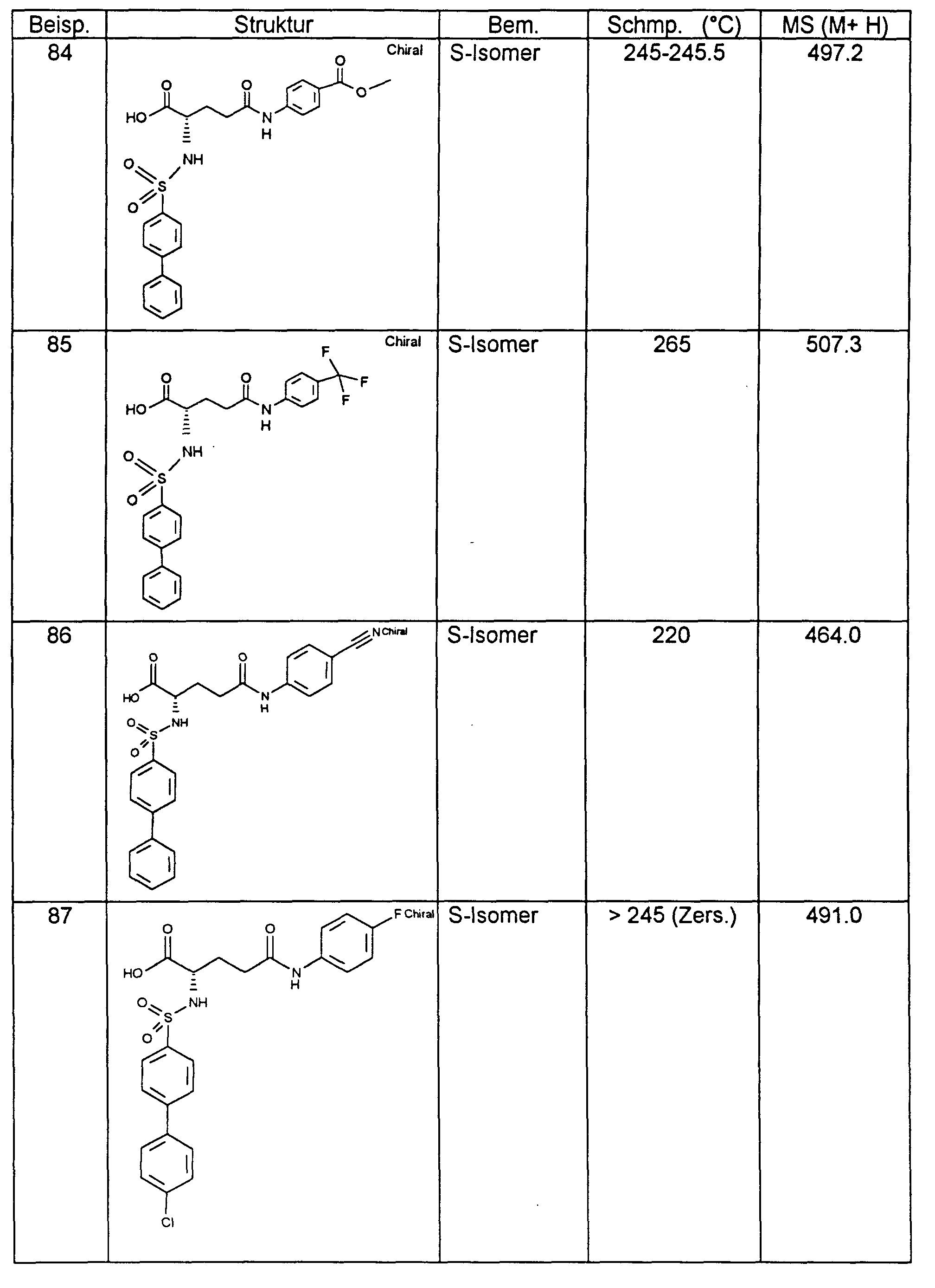

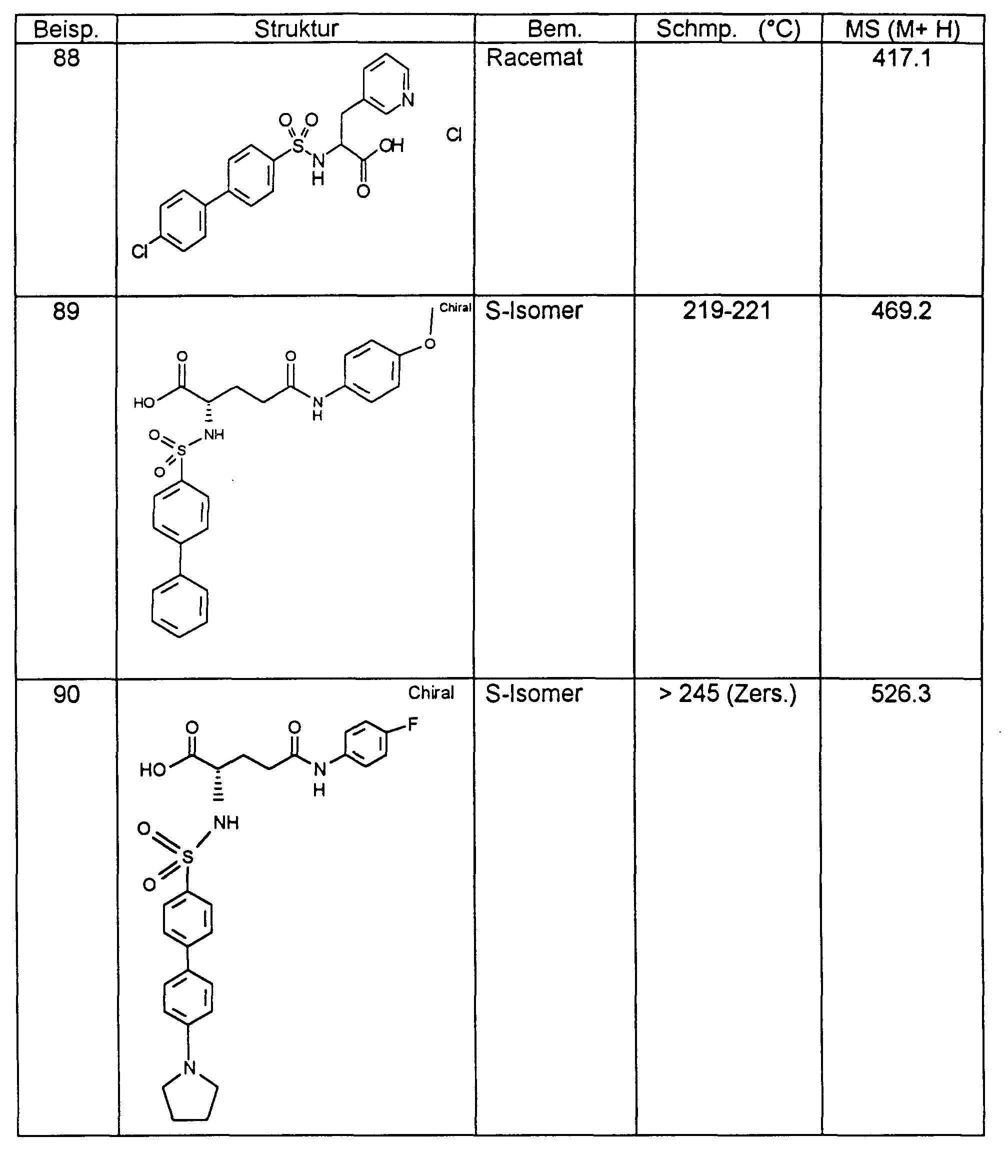

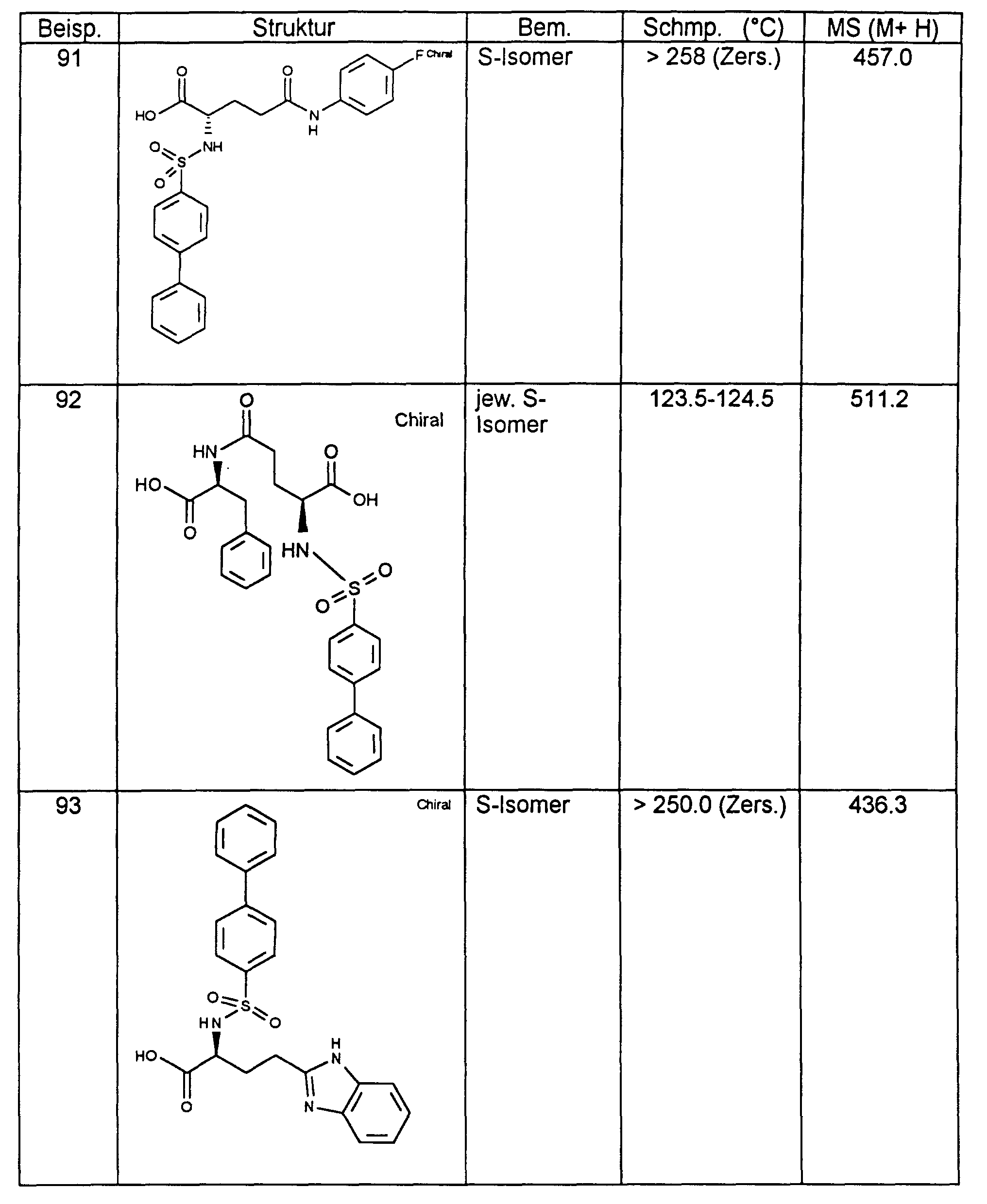

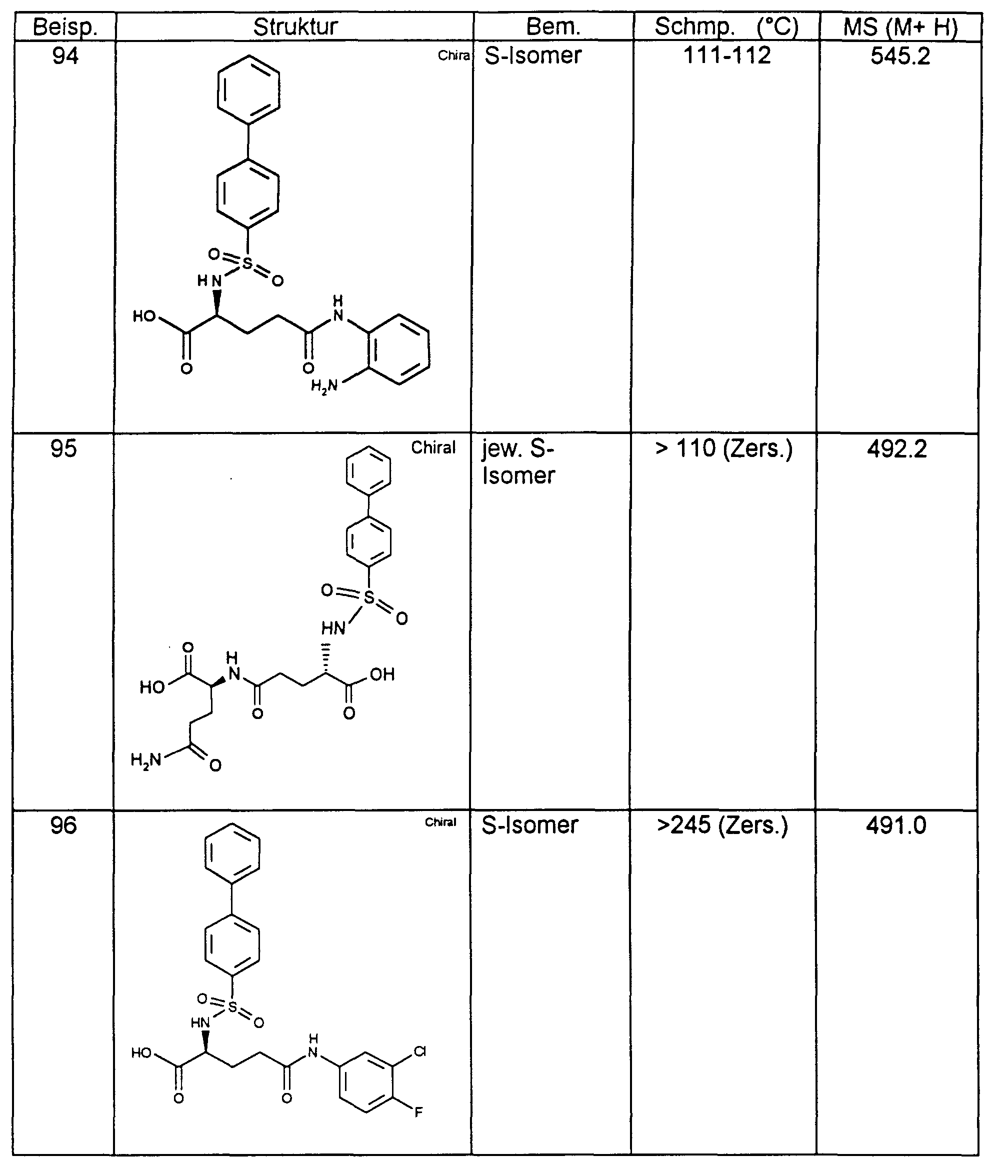

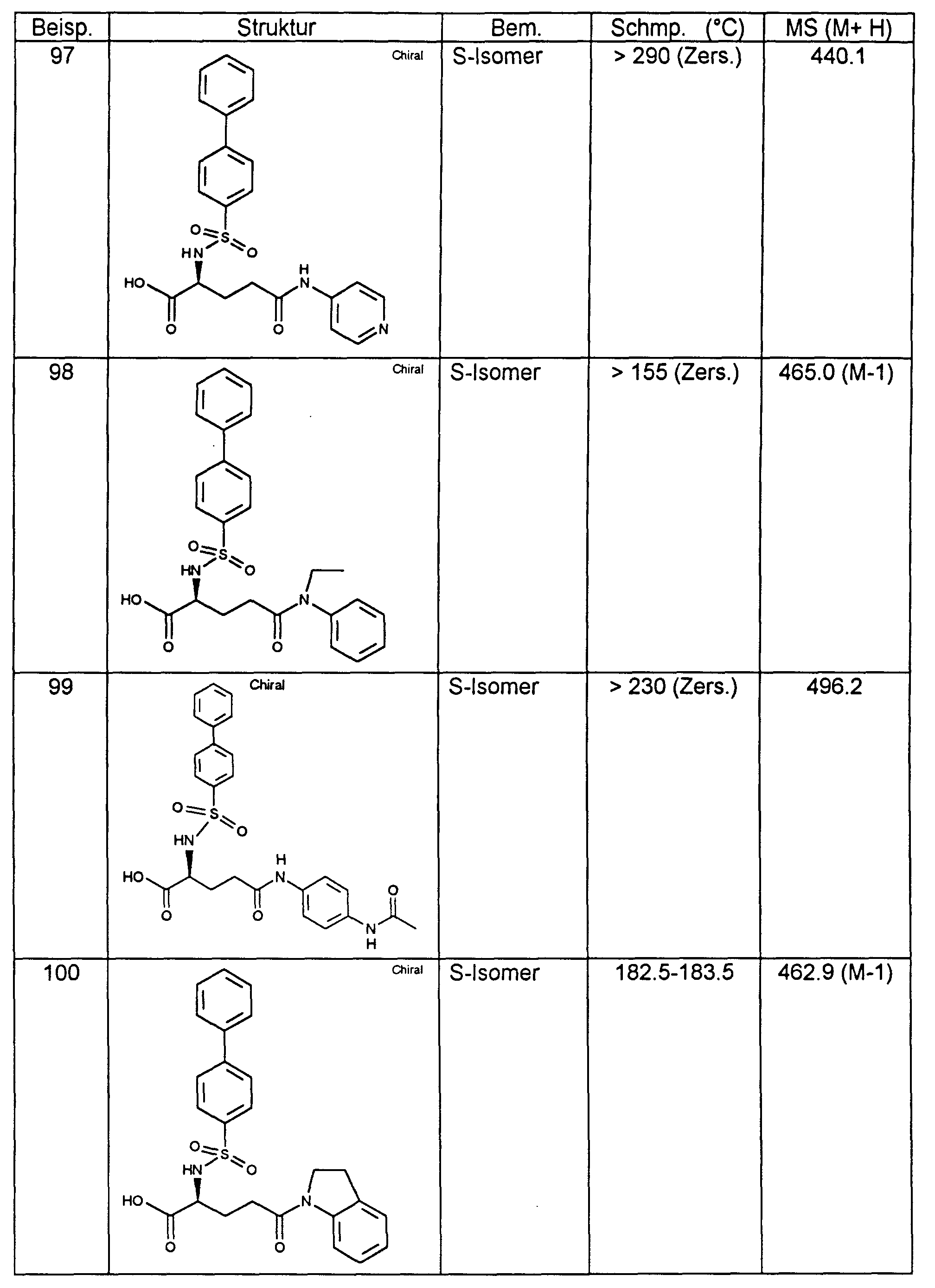

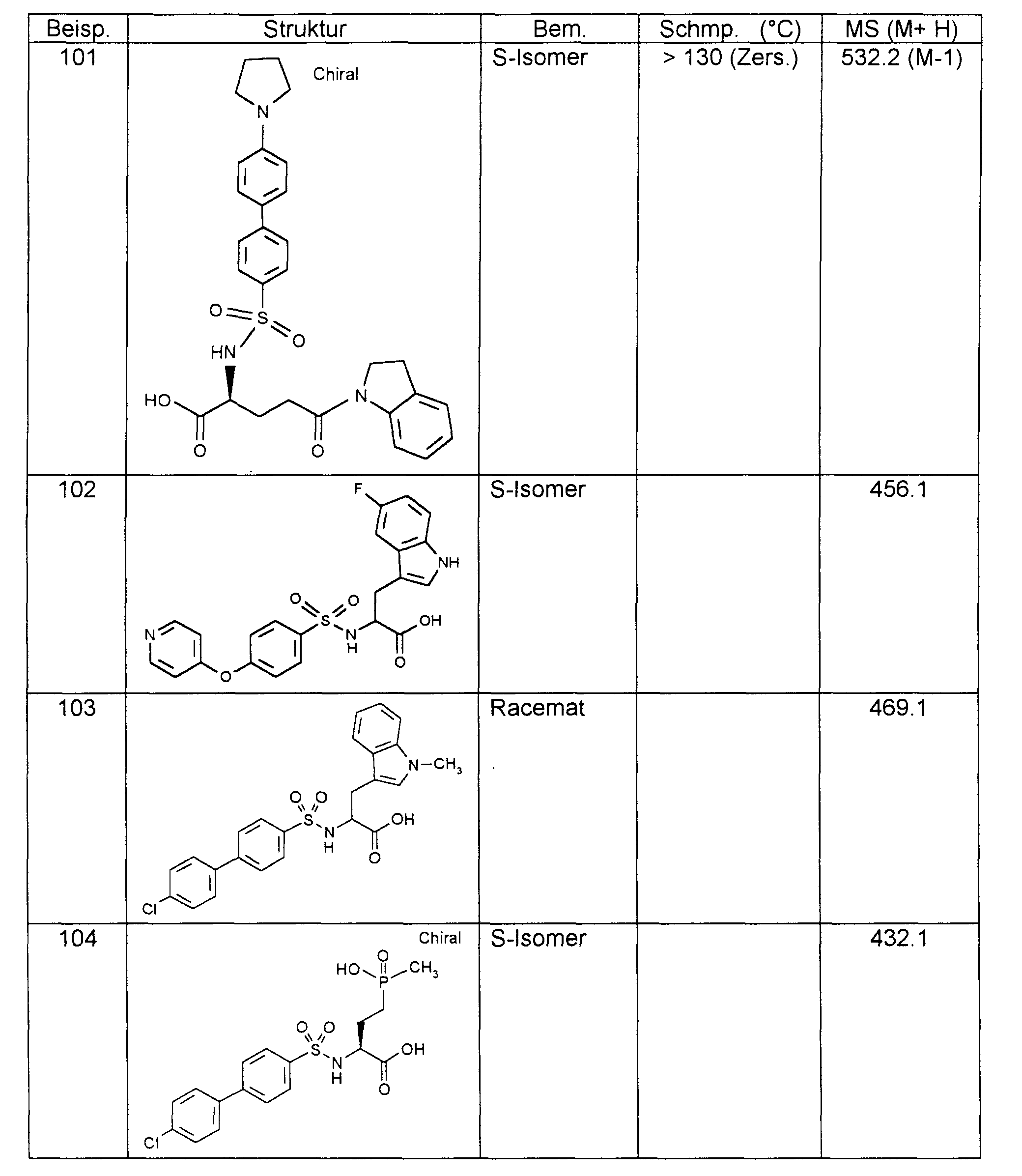

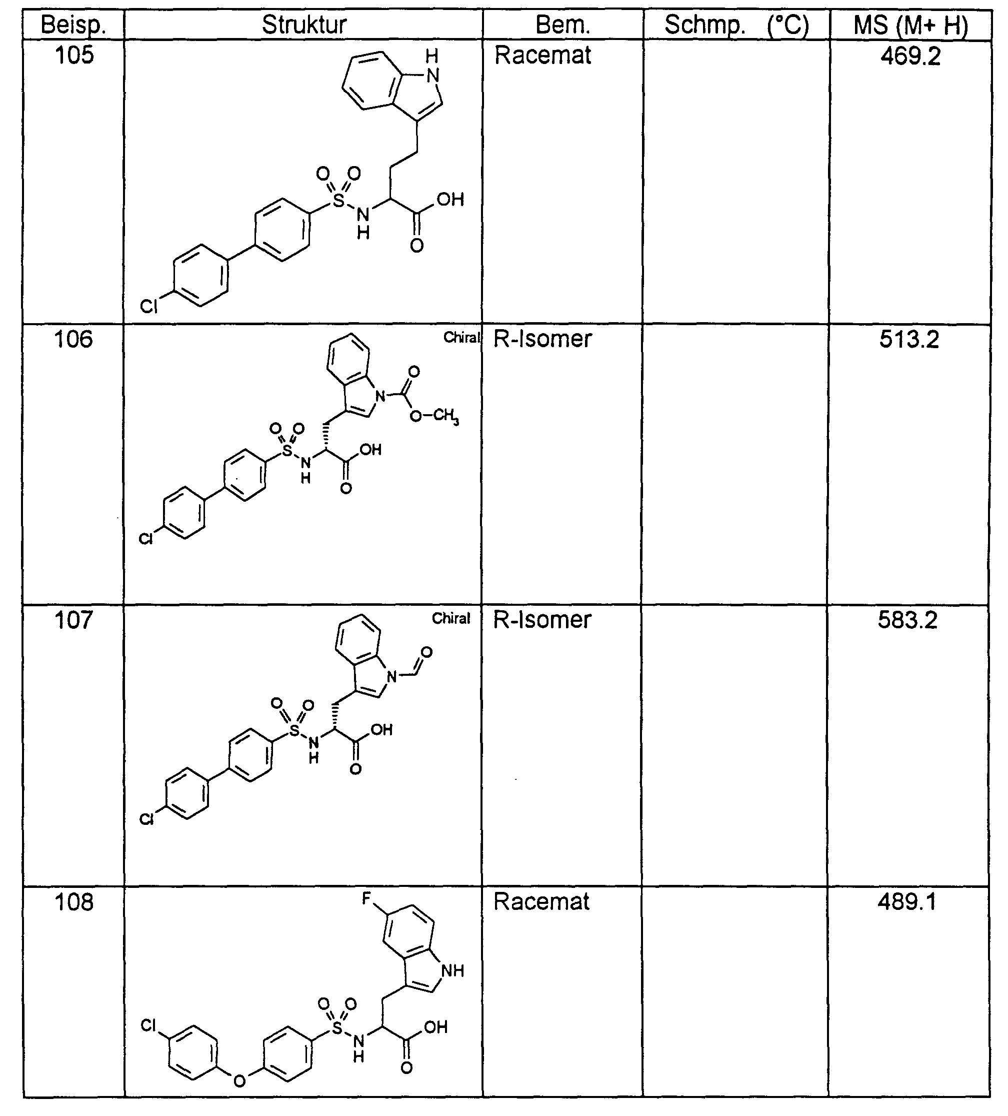

### Pharmakologische Beispiele

Darstellung und Bestimmung der enzymatischen Aktivität der katalytischen Domäne des humanen Stromelysins und der Neutrophilen-Kollagenase.

Die beiden Enzyme -Stromelysin (MMP-3) und Neutrophilen-Kollagenase (MMP-8) - wurden dargestellt nach Ye et al. (Biochemistry; 31 (1992) Seiten 11231-11235). Zur Messung der Enzymaktivität oder der Enzyminhibitorwirkung werden 70 µl Pufferlösung, und 10 µl Enzymlösung mit 10 µl einer 10%igen (v/v) wäßrigen Dimethylsulfoxid-Lösung, die gegebenenfalls den Enzyminhibitor enthält, für 15 Minuten inkubiert. Nach Zugabe von 10 µl einer 10%igen (v/v) wäßrigen Dimethylsulfoxid-Lösung, die 1 mmol/l des Substrates enthält, wird die Enzymreaktion fluoreszenzspektroskopisch verfolgt (328 nm (ex) / 393 nm(em)).
Die Enzymaktivität wird dargestellt als Extinktionszunahme/Minute. /Minute. Die in Tabelle 2 aufgeführten IC₅₀-Werte werden als diejenige Inhibitorkonzentrationen ermittelt, die jeweils zu einer 50%igen Inhibierung des Enzyms führen.
Die Pufferlösung enthält 0,05% Brij (Sigma, Deisenhofen, Deutschland) sowie 0,1 mol/l Tris/HCI, 0,1 mol/l NaCI, 0,01 mol/l CaCl₂ und 0,1 mol/l Piperazin-N,N'-bis[2-ethansulfonsäure] (pH=6,5).
Die Enzymlösung enthält 5 µg/ml einer der nach Ye et al. dargestellten Enzymdomänen. Die Substratlösung enthält 1 mmol/l des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-NH₂ (Bachem, Heidelberg, Deutschland).

**Tabelle 2**

| Beispiel Nr. | Stromelysin IC₅₀ (M) | Neutr. Kollagenase IC₅₀ (M) |
|---|---|---|
| 1 | 4*10-7 | 1*10-8 |
| 2 | 1*10-7 | 9*10-8 |
| 3 | 2*10-5 | 2*10-7 |
| 6 | 2*10-7 | 2*10-8 |
| 7 | 2*10-7 | 4*10-8 |
| 8 | 3*10-7 | 1*10-8 |
| 9 | 3*10-7 | 2*10-8 |
| 10 | 9*10-8 | 1*10-8 |
| 11 | 9*10-8 | 3*10-9 |
| 15 | 1*10-7 | 1*10-8 |
| 16 | 7*10-8 | 7*10-9 |
| 17 | 1*10-7 | 2*10-8 |
| 19 | 5*10-7 | 5*10-8 |
| 20 | 4*10-7 | 2*10-8 |
| 23 | 1*10-6 | 6*10-7 |
| 25 | 2*10-7 | 4*10-8 |
| 26 | 4*10-7 | 4*10-8 |
| 27 | 2*10-7 | 1*10-8 |
| 28 | 3*10-7 | 6*10-8 |
| 29 | 8*10-8 | 9*10-9 |
| 31 | 1*10-6 | 5*10-8 |
| 32 | 2*10-7 | 4*10-8 |
| 34 | 2*10-7 | 2*10-8 |
| 35 | 1*10-7 | 1*10-8 |
| 36 | 2*10-7 | 1*10-8 |
| 40 | 7*10-8 | 2*10-9 |
| 41 | 2*10-7 | 3*10-8 |
| 42 | 4*10-7 | 3*10-8 |
| 44 | 9*10-8 | 1*10-8 |
| 51 | 5*10-8 | 5*10-9 |
| 55 | 8*10-7 | 4*10-8 |
| 56 | 3*10-8 | 5*10-9 |
| 58 | 4*10-8 | 6*10-9 |
| 60 | 6*10-7 | 2*10-8 |
| 61 | 4*10-7 | 2*10-8 |
| 62 | 7*10-9 | 2*10-9 |
| 63 | 3*10-6 | 6*10-7 |
| 65 | 1*10-7 | 3*10-9 |
| 66 | 2*10-8 | 2*10-9 |
| 67 | 1*10-6 | 2*10-7 |
| 68 | 4*10-7 | 1*10-7 |
| 69 | 1*10-8 | 4*10-9 |
| 70 | 1*10-7 | 3*10-9 |
| 71 | 1*10-8 | 2*10-9 |
| 72 | 6*10-7 | 2*10-8 |
| 73 | 3*10-7 | 2*10-8 |
| 74 | 1*10-7 | 1*10-8 |
| 75 | 3*10-7 | 2*10-8 |
| 76 | 5*10-9 | 3*10-9 |
| 77 | 4*10-9 | 4*10-9 |
| 78 | 2*10-8 | 2*10-9 |
| 79 | 2*10-8 | 4*10-9 |
| 80 | 7*10-9 | 2*10-9 |
| 81 | 1*10-8 | 2*10-9 |
| 82 | 1*10-7 | 1*10-8 |
| 83 | 1*10-6 | 2*10-8 |
| 84 | 5*10-6 | 2*10-9 |
| 85 | 3*10-6 | 3*10-8 |
| 86 | 3*10-7 | 1*10-8 |
| 87 | 3*10-8 | 5*10-9 |
| 88 | 1*10-7 | 7*10-9 |
| 89 | 3*10-7 | 2*10-8 |
| 90 | 1*10-8 | 2*10-9 |
| 91 | 3*10-7 | 1*10-8 |
| 92 | 3*10-8 | 4*10-9 |
| 93 | 2*10-7 | 2*10-8 |
| 94 | 2*10-7 | 3*10-8 |
| 95 | 3*10-7 | 2*10-8 |
| 96 | 6*10-7 | 2*10-8 |
| 97 | 1*10-6 | 3*10-8 |
| 98 | 4*10-7 | 3*10-8 |
| 99 | 7*10-7 | 5*10-8 |
| 100 | 5*10-7 | 2*10-8 |
| 101 | 4*10-8 | 4*10-9 |
| 104 | 4*10-8 | 5*10-9 |
| 105 | 3*10-8 | 1*10-8 |
| 107 | 4*10-8 | 1*10-8 |

## Patentansprüche

1. Verbindung der Formel I 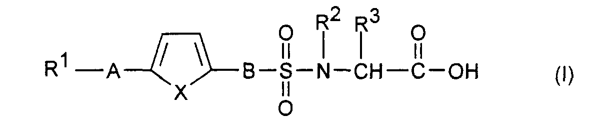 und/oder eine stereoisomere Form der Verbindung der Formel 1 und/oder ein physiologisch verträgliches Salz der Verbindung der Formel 1, wobei
R¹ für
1. Phenyl,
2. Phenyl, welches ein- oder zweifach substituiert ist durch
2.1. (C₁-C₆)-Alkyl, gerade, cyclisch oder verzweigt,
2.2. -OH,
2.3. (C₁-C₆)-Alkyl-C(O)-O-,
2.4. (C₁-C₆)-Alkyl-O-,
2.5. (C₁-C₆)-Alkyl-O-(C₁-C₄)-Alkyl-O-,
2.6. Halogen,
2.7. -CF₃,
2.8. -CN,
2.9. -NO₂,
2.10. HO-C(O)-,
2.11. (C₁-C₆)-Alkyl-O-C(O)-,
2.12. Methylendioxo,
2.13. R⁴-(R⁵)N-C(O)- oder
2.14. R⁴-(R⁵)N-, oder
3. einen Heteroaromaten aus der nachfolgenden Gruppe 3.1. bis 3.15., der unsubstituiert oder wie unter 2.1 bis 2.14 beschrieben substituiert ist,
3.1. Pyrrol,
3.2. Pyrazol,
3.3. Imidazol,
3.4. Triazol,
3.5. Thiophen,
3.6. Thiazol,
3.7. Oxazol,
3.8. Isoxazol,
3.9. Pyridin,
3.10. Pyrimidin,
3.11. Indol,
3.12 Benzothiophen,
3.13. Benzimidazol,
3.14. Benzoxazol oder
3.15. Benzothiazol steht,
R², R⁴ und R⁵ gleich oder verschieden sind und für
1. Wasserstoffatom,
2. (C₁-C₆)-Alkyl-,
3. HO-C(O)-(C₁-C₆)-Alkyl-,
4. Phenyl-(CH₂)ₙ-, wobei Phenyl unsubstituiert oder ein- oder zweifach wie unter 2.1 bis 2.14 beschrieben substituiert ist oder durch -NH-C(O)-(C₁-C₃)-Alkyl substituiert ist substituiert ist und n die ganze Zahl Null, 1 oder 2 darstellt, oder
5. Picolyl stehen oder
6. R⁴ und R⁵ zusammen mit der ringständigen Aminogruppe einen 4-bis 7-gliedrigen Ring bilden, worin gegebenenfalls eines der Kohlenstoffatome durch -O-, -S- oder -NH- ersetzt ist oder zwei benachbarte C-Atome des 4- bis 7-gliedrigen Rings Teil eines Benzylrestes sind,
R³ für
1. (C₂-C₁₀)-Alkenyl-, worin Alkenyl gerade oder verzweigt ist,
2. R²-S(O)ₙ-(C₁-C₆)-Alkyl-, wobei n die obengenannte Bedeutung hat und worin R² die obengenannte Bedeutung für 2. bis 5. hat,
3. R²-S(O)(=NH)-(C₁-C₆)-Alkyl-, worin R² die obengenannte Bedeutung für 2. bis 5. hat,
4. 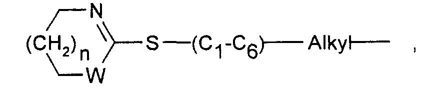 worin n die ganze Zahl Null, 1 oder 2 darstellt und W ein Stickstoff-, Sauerstoff- oder Schwefelatom darstellt,
5. -(CH₂)ₘ-P(O)(OH)-(C₁-C₃)-Alkyl, worin m die ganze Zahl Null, 1, 2, 3, 4, 5 oder 6 darstellt, oder
6. R⁶-C(O)-(C₁-C₆)-Alkyl- steht, worin R⁶ für
1. (C₁-C₆)-Alkyl-,
2. HO-C(O)-CH(R³)-NH-, worin R³ für
2.1. Wasserstoffatom,
2.2. (C₁-C₁₀)-Alkyl-, worin Alkyl unsubstituiert ist, und/oder ein Wasserstoffatom des Alkylrestes durch -OH ersetzt ist,
2.3. (C₂-C₁₀)-Alkenyl-, worin Alkenyl gerade oder verzweigt ist,
2.4. R²-O-(C₁-C₆)-Alkyl-, worin R² die obengenannte Bedeutung für 1. bis 5. hat,
2.5. R²-S(O)ₙ-(C₁-C₆)-Alkyl-, wobei n die ganze Zahl Null, 1 oder 2 darstellt und worin R² die obengenannte Bedeutung für 1. bis 5. hat,
2.6. R²-S(O)(=NH)-(C₁-C₆)-Alkyl-, worin R² die obengenannte Bedeutung für 1. bis 5. hat,
2.7. 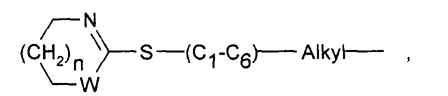 worin n die ganze Zahl Null, 1 oder 2 darstellt und W ein Stickstoff-, Sauerstoff- oder Schwefelatom darstellt,
2.8. Phenyl-(CH₂)ₘ-, worin m die ganze Zahl Null, 1, 2, 3, 4, 5 oder 6 ist und/oder ein Wasserstoffatom der -(CH₂)ₘ-Kette durch -OH ersetzt ist und Phenyl unsubstituiert oder einoder zweifach substituiert ist mit
2.8.1 wie oben unter 2.1. bis 2.14. beschrieben,
2.8.2 -O-(CH₂)ₘ-Phenyl, worin Phenyl unsubstituiert oder ein- oder zweifach wie oben unter 2.1. bis 2.14. beschrieben substituiert ist und m die ganze Zahl Null, 1, 2, 3, 4, 5 oder 6 darstellt, oder
2.8.3 -C(O)-(CH₂)ₘ-Phenyl, worin Phenyl und m wie unter 2.8.2 definiert ist,
2.9. Heteroaryl-(CH₂)ₘ-, worin Heteroaryl wie unter 3.1. bis 3.15. definiert ist, m die ganze Zahl Null, 1, 2, 3, 4, 5 oder 6 darstellt wie oben definiert ist und/oder ein Wasserstoffatom der -(CH₂)ₘ-Kette durch -OH ersetzt ist und Heteroaryl unsubstituiert oder ein- oder zweifach substituiert ist mit
2.9.1 wie oben unter 2.1 bis 2.14 beschrieben,
2.9.2 -CH(O),
2.9.3 -SO₂-Phenyl, worin Phenyl unsubstituiert oder wie unter 2.8.2 oder 2.8.3 definiert ist, oder
2.9.4 -O-(CH₂)ₘ-Phenyl, worin m die ganze Zahl Null, 1, 2, 3, 4, 5 oder 6 ist, oder
2.10. -(CH₂)ₘ-P(O)(OH)-(C₁-C₃)-Alkyl, worin m die ganze Zahl Null, 1, 2, 3, 4, 5 oder 6 ist,
3. Phenyl, worin Phenyl unsubstituiert oder wie unter 2.1. bis 2.14.beschrieben substituiert ist,
4. Heteroaryl, worin Heteroaryl wie unter 3.1. bis 3.15. definiert und/oder wie unter 2.1 bis 2.14 beschrieben substituiert ist,
5. R⁴-(R⁵)N-NH-, worin R⁴ und R⁵ wie oben definiert sind,
6. R²O-, worin R² die obengenannte Bedeutung für 2. bis 5. hat,
7. R⁴-(R⁵ )N- bedeutet, worin R⁴ und R⁵ die obengenannte Bedeutung für 1. bis 5. haben, aber nicht gleichzeitig Wasserstoffatom sind, oder
8. Heteroaryl-(CH₂)ₘ-NH-, worin Heteroaryl wie unter 3.1. bis 3.15. definiert und/oder wie unter 2.1. bis 2.14 beschrieben substituiert ist und m die ganze Zahl Null, 1, 2, 3, 4, 5 oder 6 darstellt oder durch -(C₁-C₄)-Alkyl-COOH substituiert ist, steht, oder
R² und R³ bilden zusammen einen Ring mit ringständiger Carboxylgruppe der
Teilformel II, 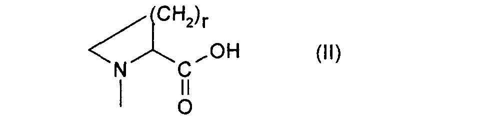 worin r die ganze Zahl Null, 1, 2 oder 3 darstellt und/oder eines der Kohlenstoffatome im Ring durch -O-, -S- oder -(R⁷)N- ersetzt ist, worin
R⁷ für
1. Wasserstoffatom,
2. (C₁-C₆)-Alkyl,
3. Phenyl, worin Phenyl unsubstituiert oder wie unter 2.1 bis 2.14 beschrieben substituiert ist,
4. Benzyl, worin Benzyl unsubstituiert oder wie unter 2.1 bis 2.14 beschrieben substituiert ist, oder
5. R²N-C(=NH)- steht, wobei R² die obengenannte Bedeutung für 1. bis 5. hat,
und/oder die Kohlenstoffatome im Ring der Teilformel II ein- oder mehrfach durch (C₁-C₆)-Alkyl-, Phenyl-, Phenyl-(CH₂)ₘ- oder -OH substituiert sind, worin m die ganze Zahl Null, 1, 2, 3, 4, 5 oder 6 darstellt,
A für
a) eine kovalente Bindung,
b) -O-,
c) -CH=CH- oder
d) -C≡C- steht,
B für
a) -(CH₂)ₘ-, worin m die ganze Zahl Null, 1, 2, 3, 4, 5 oder 6 darstellt,
b) -O-(CH₂)ₚ, worin p eine ganze Zahl von 1 bis 5 bedeutet, oder
c) -CH=CH- steht und
X für -CH=CH-, Sauerstoffatom oder Schwefelatom steht.

2. Verbindung der Formel I gemäß Anspruch 1, **dadurch gekennzeichnet, daß**
R¹ für
1. Phenyl oder
2. Phenyl, welches einfach substituiert ist durch
2.1. (C₁-C₆)-Alkyl-, worin Alkyl gerade, cyclisch oder verzweigt ist,
2.2. -OH,
2.3. (C₁-C₆)-Alkyl-C(O)-O-,
2.4. (C₁-C₆)-Alkyl-O-,
2.5. (C₁-C₆)-Alkyl-O-(C₁-C₄)-Alkyl-O-,
2.6. Halogen,
2.7. -CF₃ oder
2.8. R⁴-(R⁵ )N- steht,
R², R⁴ und R⁵ gleich oder verschieden sind und für
1. Wasserstoffatom oder
2. (C₁-C₆)-Alkyl- stehen,
R³ für
1. R²-S(O)ₙ-(C₁-C₆)-Alkyl-, worin R² (C₁-C₆)-Alkyl- oder Phenyl-(CH₂)ₙbedeutet und n die ganze Zahl Null oder 1 ist, oder
2. R⁶-C(O)-(C₁-C₆)-Alkyl- steht, worin
R⁶ für
1. R²O-, worin R² Wasserstoffatom oder (C₁-C₆)-Alkylbedeutet, oder
2. R⁴-(R⁵)N- steht, worin R⁴ und R⁵ die obengenannte Bedeutung für 1. bis 2. haben, aber nicht gleichzeitig Wasserstoffatom sind, oder R⁴ und R⁵ zusammen mit der ringständigen Aminogruppe einen 5- bis 6-gliedrigen Ring bilden, worin gegebenenfalls eines der Kohlenstoffatome durch -O-, -S- oder -NH- ersetzt ist oder zwei benachbarte C-Atome des 5- bis 6- gliedrigen Rings Teil eines Benzylrestes sind, oder
R² und R³ bilden zusammen einen Ring mit ringständiger Carboxylgruppe der Teilformel II, worin n die ganze Zahl 1 oder 2 bedeutet und/oder eines der Kohlenstoffatome im Ring durch -O- oder -(R⁷)N- ersetzt ist, und
R⁷ für
1. Wasserstoffatom,
2. (C₁-C₆)-Alkyl,
3. Phenyl, worin Phenyl unsubstituiert oder wie oben 2.1 bis 2.14 beschrieben substituiert ist,
4. Benzyl, worin Benzyl unsubstituiert oder wie oben 2.1 bis 2.14 beschrieben substituiert ist, oder
5. R²N-C(=NH)- steht, worin R² (C₁-C₆)-Alkyl- bedeutet, und/oder die Kohlenstoffatome im Ring der Teilformel II mit Phenyl oder -OH einfach substituiert sind,
A für
a) eine kovalente Bindung oder
b) -O- steht,
B für
a) -(CH₂)ₘ-, worin m die ganze Zahl Null, 1 oder 2 bedeutet, oder
b) -O-(CH₂)ₚ, worin p eine ganze Zahl von 1 oder 2 bedeutet, steht
und X für -CH=CH- steht.

3. Verbindung der Formel I, gemäß Anspruch 1, **dadurch gekennzeichnet, daß**
R¹ für
1. Phenyl oder
2. Phenyl, welches einfach substituiert ist durch
2.1. Halogen, insbesondere Chlor oder Fluor oder
2.2. R⁴-(R⁵)N-, wobei R⁴ und R⁵ gleich oder verschieden sind und für
2.2.1. (C₁-C₃)-Alkyl oder
2.2.2. R⁴ und R⁵ zusammen mit der ringständigen Aminogruppe einen 5-6-gliedrigen Ring bilden, wobei gegebenenfalls eines der C-Atome durch -O- oder -N- ersetzt ist,
stehen,
R² für Wasserstoffatom
R³ für
1. R⁶-C(O)-(C₂-C₃)-Alkyl steht, worin
R⁶ für
1. R⁴-(R⁵)N- steht, worin R⁴ und R⁵ gleich oder verschieden sind, aber nicht gleichzeitig Wasserstoffatom sind und für
1.1. Wasserstoffatom,
1.2. (C₁-C₃)-Alkyl-,
1.3. Phenyl-(CH₂)ₙ-, wobei Phenyl unsubstituiert oder ein oder zweifach wie unter 2.1 bis 2.14 beschrieben substituiert ist und n die ganze Zahl Null, 1, oder 2 darstellt,
1.4. R⁴ und R⁵ zusammen mit der ringständigen Aminogruppe einen 5-6-gliedrigen Ring bilden, wobei gegebenenfalls eines der C-Atome durch -O- oder -NHersetzt ist oder einen Indolin-Rest bilden, oder
1.5. HO-C(O)-CH(R³)-NH-, worin R³ Heteroaryl-(CH₂)ₘ-, worin Heteroaryl wie oben unter 3.5, 3.11 oder 3.13 definiert ist und das Heteroaryl unsubstituiert oder einfach substituiert ist wie unter 2.1 bis 2.14 beschrieben und m die ganze Zahl 1 oder 2 bedeutet,
A für eine kovalente Bindung steht,
B für -(CH₂)ₒ- steht, worin o Null bedeutet und
X für -CH=CH- steht.

4. Verbindung der Formel VI 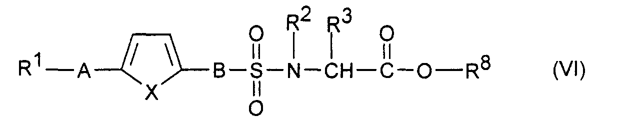 und/oder eine stereoisomere Form der Verbindung der Formel VI und/oder ein physiologisch verträgliches Salz der Verbindung der Formel VI, wobei R¹, A, X, B, R² und R³ die in der Verbindung der Formel I gemäß Anspruch 1 genannte Bedeutung haben und R⁸ Wasserstoffatom, (C₁-C₆)-Alkyl, Phenyl oder Benzyl bedeutet.

5. Verfahren zur Herstellung der Verbindung der Formel I gemäß einem oder mehreren der Ansprüche 1 bis 4, **dadurch gekennzeichnet, daß** man
a) eine Aminocarbonsäure der Formel III, 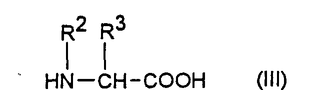 worin R² und R³ wie in Formel I definiert sind, mit einem Sulfonsäurederivat der Formel IV, 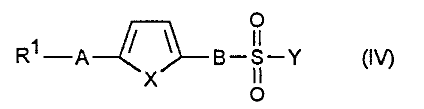 worin R¹, A und B wie in Formel I definiert sind und Y ein Halogenatom, Imidazolyl oder -OR⁸ bedeutet, worin R⁸ Wasserstoffatom, (C₁-C₆)-Alkyl, Phenyl oder Benzyl, gegebenenfalls substituiert darstellt, in Gegenwart einer Base oder gegebenenfalls eines wasserentziehenden Mittels zu einer Verbindung der Formel I umsetzt wird, oder
b) einen Aminocarbonsäureester der Formel V, 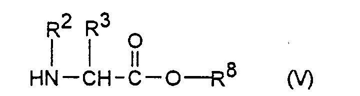 worin R², R³ und R⁸ die obengenannte Bedeutung haben, mit einem Sulfonsäurederivat der Formel IV unter den obengenannten Bedingungen zu einer Verbindung der Formel VI 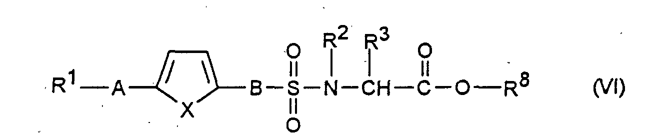 umsetzt, und die Verbindung der Formel VI unter Abspaltung des Restes R⁸, bevorzugt in Gegenwart einer Base oder Säure in eine Verbindung der Formel I umwandelt, oder
c) die Verbindung der Formel VII, 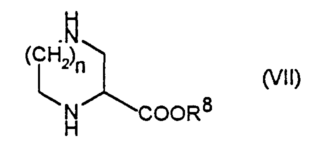 worin n die ganze Zahl Null, 1 oder 2 darstellt, mit Hilfe einer Schutzgruppe E zu einer Verbindung der Formel VIII umsetzt, 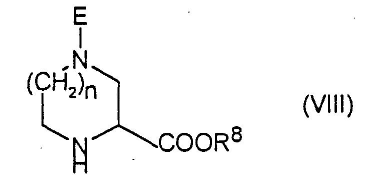 die Verbindung der Formel VIII mit dem Sulfonsäurederivat der Formel IV unter den obengenannten Bedingungen in eine Verbindung der Formel IX überführt 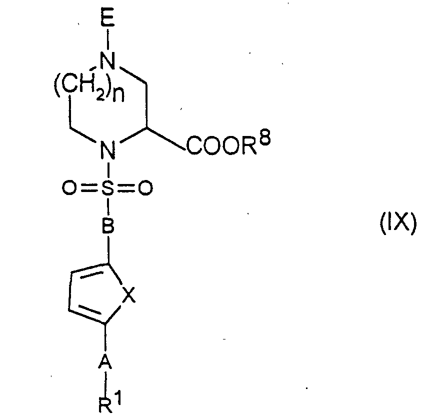 und anschließend die Verbindung der Formel IX unter Abspaltung der Schutzgruppe E und des Restes R⁸ mit Hilfe geeigneter Spaltreagenzien in die Verbindung der Formel I überführt, oder
d) eine nach den Verfahren a), b) oder c) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
e) die nach den Verfahren a), b), c) oder d) hergestellte Verbindung der Formel I entweder in freier Form isoliert oder im Falle des Vorliegens von sauren oder basischen Gruppen in physiologisch verträgliche Salze umwandelt.

6. Arzneimittel, **gekennzeichnet durch** einen wirksamen Gehalt an mindestens einer Verbindung der Formel I gemäß einem oder mehreren der Ansprüche 1 bis 4 zusammen mit einem pharmazeutisch geeigneten und physiologisch verträglichen Trägerstoff, Zusatzstoff und/oder anderen Wirk- und Hilfsstoffen.

7. Verwendung von mindestens einer Verbindung der Formel I gemäß einem oder mehreren der Ansprüche 1 bis 4, zur Herstellung von Arzneimitteln zur Prophylaxe und Therapie von Erkrankungen, an deren Verlauf eine verstärkte Aktivität von Matrix-abbauenden Metalloproteinasen beteiligt ist.

8. Verwendung gemäß Anspruch 7, für die Behandlung von degenerativen Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen, Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels, der Ulceration, Atherosklerose und Stenosen, aber auch zur Behandlung von Entzündungen, Krebserkrankungen, Tumormetastasenbildung, Kachexie, Anorexie und septischem Schock.

9. Verfahren zur Herstellung eines Arzneimittels , **dadurch gekennzeichnet, daß** man mindestens eine Verbindung der Formel I gemäß einem oder mehreren der Ansprüche 1 bis 4 mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

## Claims

1. A compound of the formula I 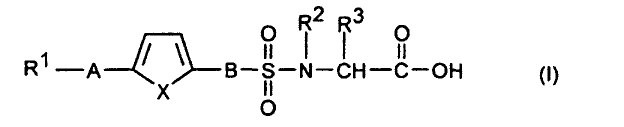 and/or a stereoisomeric form of the compound of the formula I and/or a physiologically tolerable salt of the compound of the formula I, where
R¹ is
1. phenyl,
2. phenyl, which is mono- or disubstituted by
2.1. (C₁-C₆)-alkyl, which is linear, cyclic or branched,
2.2. -OH,
2.3. (C₁-C₆)-alkyl-C(O)-O-,
2.4. (C₁-C₆)-alkyl-O-,
2.5. (C₁-C₆)-alkyl-O-(C₁-C₄)-alkyl-O-,
2.6. halogen,
2.7. -CF₃,
2.8. -CN,
2.9. -NO2,
2.10. HO-C(O)-,
2.11. (C₁-C₆)-alkyl-O-C(O)-,
2.12. methylenedioxo,
2.13. R⁴-(R⁵)N-C(O)- or
2.14. R⁴-(R⁵)N-, or
3. a heteroaromatic from the following group 3.1. to 3.15., which is unsubstituted or substituted as described under 2.1 to 2.14,
3.1. pyrrole,
3.2. pyrazole,
3.3. imidazole,
3.4. triazole,
3.5. thiophene,
3.6. thiazole,
3.7. oxazole,
3.8. isoxazole,
3.9. pyridine,
3.10. pyrimidine,
3.11. indole,
3.12. benzothiophene,
3.13. benzimidazole,
3.14. benzoxazole or
3.15. benzothiazole,
R², R⁴ and R⁵ are identical or different and are
1. a hydrogen atom,
2. (C₁-C₆)-alkyl-,
3. HO-C(O)-(C₁-C₆)-alkyl-,
4. phenyl-(CH₂)ₙ-, in which phenyl is unsubstituted or mono- or disubstituted as described under 2.1 to 2.14 or is substituted by -NH-C(O)-(C₁-C₃)-alkyl and n is the integer zero, 1 or 2, or
5. picolyl or
6. R⁴ and R⁵ together with the ring amino group form a 4- to 7-membered ring, in which one of the carbon atoms is optionally replaced by -O-, -S- or -NH- or two adjacent carbon atoms of the 4- to 7-membered ring are part of a benzyl radical,
R³ is
1. (C₂-C₁₀)-alkenyl-, in which alkenyl is linear or branched,
2. R²-S(O)ₙ-(C₁-C₆)-alkyl-, where n has the abovementioned meaning and R² has the abovementioned meaning for 2. to 5.,
3. R²-S(O)(=NH)-(C₁-C₆)-alkyl-, in which R² has the abovementioned meaning for 2. to 5.,
4. 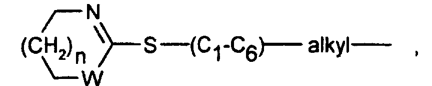 in which n is the integer zero, 1 or 2 and W is a nitrogen, oxygen or sulfur atom,
5. -(CH₂)ₘ-P(O)(OH)-(C₁-C₃)-alkyl, in which m is the integer zero, 1, 2, 3, 4, 5, or 6, or
6. R⁶-C(O)-(C₁-C₆)-alkyl-, in which R⁶ is
1. (C₁-C₆)-alkyl-,
2. HO-C(O)-CH(R³)-NH-, in which R³ is
2.1. a hydrogen atom,
2.2. (C₁-C₁₀)-alkyl, in which alkyl is unsubstituted, and/or a hydrogen atom of the alkyl radical is replaced by -OH,
2.3. (C₂-C₁₀)-alkenyl, in which alkenyl is linear or branched,
2.4. R²-O-(C₁-C₆)-alkyl, in which R² has the abovementioned meaning for 1. to 5.,
2.5. R²-S(O)ₙ-(C₁-C₆)-alkyl, where n is the integer zero, 1 or 2 and in which R² has the abovementioned meaning for 1. to 5.,
2.6. R²-S(O)(=NH)-(C₁-C₆)-alkyl-, in which R² has the abovementioned meaning for 1. to 5.,
2.7. 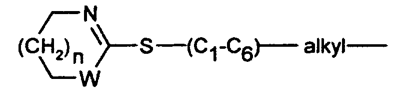 in which n is the integer zero, 1 or 2 and W is a nitrogen, oxygen or sulfur atom,
2.8. phenyl-(CH₂)ₘ-, in which m is the integer zero, 1, 2, 3, 4, 5 or 6 and/or a hydrogen atom of the-(CH₂)ₘ- chain is replaced by -OH and phenyl is unsubstituted or mono- or disubstituted by
2.8.1 as described under 2.1. to 2.14.,
2.8.2 -O-(CH₂)ₘ-phenyl, in which phenyl is unsubstituted or mono- or disubstituted as described under 2.1. to 2.14. and m is the integer zero, 1, 2, 3, 4, 5 or 6, or
2.8.3 -C(O)-(CH₂)ₘ-phenyl, in which phenyl and m are as defined under 2.8.2,
2.9. heteroaryl-(CH₂)ₘ-, in which heteroaryl is as defined under 3.1. to 3.15., m is the integer zero, 1, 2, 3, 4, 5 or 6 as defined above and/or a hydrogen atom of the -(CH₂)ₘ-chain is replaced by -OH and heteroaryl is unsubstituted or mono- or disubstituted by
2.9.1 as described under 2.1 to 2.14,
2.9.2 -CH(O),
2.9.3 -SO₂-phenyl, in which phenyl is unsubstituted or is as defined under 2.8.2 or 2.8.3, or
2.9.4 -O-(CH2)ₘ-phenyl, in which m is the integer zero, 1, 2, 3, 4, 5 or 6, or
2.10. -(CH₂)ₘ-P(O)(OH)-(C₁-C₃)-alkyl, in which m is the integer zero, 1, 2, 3, 4, 5 or 6,
3. phenyl, in which phenyl is unsubstituted or substituted as described under 2.1 to 2.14,
4. heteroaryl, in which heteroaryl is as defined under 3.1. to 3.15. and/or is substituted as described under 2.1 to 2.14,
5. R⁴-(R⁵)N-NH-, in which R⁴ and R⁵ are as defined above,
6. R²O-, in which R² has the abovementioned meaning for 2. to 5.,
7. R⁴-(R⁵ )N-, in which R⁴ and R⁵ have the abovementioned meaning for 1. to 5. but are not simultaneously hydrogen atoms, or,
8. heteroaryl-(CH₂)ₘ-NH-, in which heteroaryl is as defined under 3.1 to 3.15. and/or is substituted as described under 2.1. to 2.14 and m is the integer zero, 1, 2, 3, 4, 5 or 6 or is substituted by -(C₁-C₄)-alkyl-COOH, or
R² and R³ together form a ring having a ring carboxyl group, of the subformula II 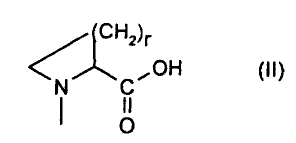
in which r is the integer zero, 1, 2 or 3 and/or one of the carbon atoms in the ring is replaced by -O-, -S- or -(R⁷)N-, in which
R⁷ is
1. a hydrogen atom,
2. (C₁-C₆)-alkyl,
3. phenyl, in which phenyl is unsubstituted or is substituted as described under 2.1 to 2.14,
4. benzyl, in which benzyl is unsubstituted or substituted as described under 2.1 to 2.14, or
5. R²N-C(=NH)-, where R² has the abovementioned meaning for 1. to 5.,
and/or the carbon atoms in the ring of the subformula II are mono- or polysubstituted by (C₁-C₆)-alkyl-, phenyl-, phenyl-(CH₂)ₘ- or -OH, in which m is the integer zero, 1, 2, 3, 4, 5 or 6,
A is
a) a covalent bond,
b) -O-,
c) -CH=CH- or
d) -C≡C-,
B is
a) -(CH₂)ₘ-, in which m is the integer zero, 1, 2, 3, 4, 5 or 6,
b) -O-(CH₂)ₚ, in which p is an integer from 1 to 5, or
c) -CH=CH- and
X is -CH=CH-, an oxygen atom or a sulfur atom.

2. A compound of the formula I as claimed in claim 1, wherein
R¹ is
1. phenyl or
2. phenyl which is monosubstituted by
2.1. (C₁-C₆)-alkyl-, in which alkyl is linear, cyclic or branched,
2.2. -OH,
2.3. (C₁-C₆)-alkyl-C(O)-O-,
2.4. (C₁-C₆)-alkyl-O-,
2.5. (C₁-C₆)-alkyl-O-(C₁-C₄)-alkyl-O-,
2.6. halogen,
2.7. -CF₃ or
2.8. R⁴-(R⁵)N-,
R², R⁴ and R⁵ are identical or different and are
1. a hydrogen atom or
2. (C₁-C₆)-alkyl-,
R³ is
1. R²-S(O)ₙ-(C₁-C₆)-alkyl-, in which R² (C₁-C₆)-alkyl- or phenyl-(CH₂)ₙ- and n is the integer zero or 1, or
2. R⁶-C(O)-(C₁-C₆)-alkyl-, in which
R⁶ is
1. R²O-, in which R² is a hydrogen atom or (C₁-C₆)-alkyl, or
2. R⁴-(R⁵)N-, in which R⁴ and R⁵ have the abovementioned meaning for 1. to 2. but are not simultaneously hydrogen atoms, or R⁴ and R⁵ together with the ring amino group form a 5- to 6-membered ring in which one of the carbon atoms is optionally replaced by -O-, -S- or -NH- or two adjacent carbon atoms of the 5- to 6-membered ring are part of a benzyl radical, or
R² and R³ together form a ring having a ring carboxyl group, of the subformula II, in which n is the integer 1 or 2 and/or one of the carbon atoms in the ring is replaced by -O- or -(R⁷)N-, and
R⁷ is
1. a hydrogen atom,
2. (C₁-C₆)-alkyl,
3. phenyl, in which phenyl is unsubstituted or substituted as described above under 2.1 to 2.14,
4. benzyl, in which benzyl is unsubstituted or substituted as described above under 2.1 to 2.14, or
5. R²N-C(=NH)-, in which R² is (C₁-C₆)-alkyl-, and/or the carbon atoms in the ring of the subformula II are monosubstituted by phenyl or-OH,
A is
a) a covalent bond or
b) -O-,
B is
a) -(CH₂)ₘ-, in which m is the integer zero, 1 or 2, or
b) -O-(CH2)p, in which p is an integer 1 or 2, and
X is -CH=CH-.

3. A compound of the formula I as claimed in claim 1, wherein
R¹ is
1. phenyl or
2. phenyl which is monosubstituted by
2.1. halogen, in particular chlorine or fluorine or
2.2. R⁴-(R⁵)N-, in which R⁴ and R⁵ are identical or different and are
2.2.1. (C₁-C₃)-alkyl or
2.2.2. R⁴ and R⁵ together with the ring amino group form a 5- to 6-membered ring, one of the carbon atoms optionally being replaced by -O- or -N-,
R² is a hydrogen atom,
R³ is
1. R⁶-C(O)-(C₂-C₃)-alkyl, in which
R⁶ is
1. R⁴-(R⁵)N-, in which R⁴ and R⁵ are identical or different but are not simultaneously hydrogen atoms and are
1.1. a hydrogen atom,
1.2. (C₁-C₃)-alkyl-,
1.3. phenyl-(CH₂)ₙ-, where phenyl is unsubstituted or mono- or disubstituted as described under 2.1 to 2.14 and n is the integer zero, 1 or 2,
1.4. R⁴ and R⁵ together with the ring amino group form a 5- to 6-membered ring, where one of the carbon atoms is optionally replaced by-O- or-NH-, or form an indoline radical, or
1.5. HO-C(O)-CH(R³)-NH-, in which R³ is heteroaryl-(CH₂)ₘ-, in which heteroaryl is as defined above under 3.5, 3.11 or 3.13 and the heteroaryl is unsubstituted or monosubstituted as described under 2.1 to 2.14 and m is the integer 1 or 2,
A is a covalent bond,
B is -(CH₂)ₒ-, in which o is zero and
X is -CH=CH-.

4. A compound of the formula VI 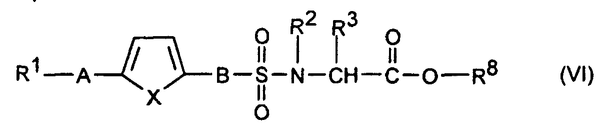 and/or a stereoisomeric form of the compound of the formula VI and/or a physiologically tolerable salt of the compound of the formula VI, R¹, A, X, B, R² and R³ having the meaning mentioned in the compound of the formula I as claimed in claim 1 and R⁸ being a hydrogen atom, (C₁-C₆)-alkyl, phenyl or benzyl.

5. A process for the preparation of the compound of the formula I as claimed in one or more of claims 1 to 4, which comprises
a) reacting an aminocarboxylic acid of the formula III, 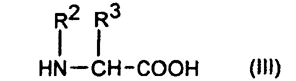 in which R² and R³ are as defined in formula I, with a sulfonic acid derivative of the formula IV, 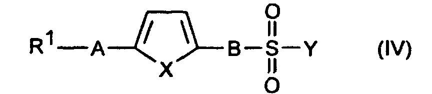 in which R¹, A and B are as defined in formula I and Y is a halogen atom, imidazolyl or -OR⁸, in which R⁸ is a hydrogen atom, (C₁-C₆)-alkyl, phenyl or benzyl, if appropriate substituted, in the presence of a base or optionally of a dehydrating agent to give a compound of the formula I, or
b) reacting an aminocarboxylic acid ester of the formula V, 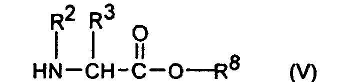 in which R², R³ and R⁸ have the abovementioned meaning, with a sulfonic acid derivative of the formula IV under the abovementioned conditions to give a compound of the formula VI 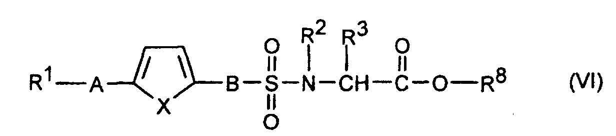 and converting the compound of the formula VI into a compound of the formula I with removal of the radical R⁸, preferably in the presence of a base or acid, or
c) reacting the compound of the formula VII, 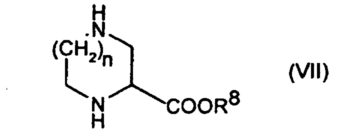 where n is the integer zero, 1 or 2, with the aid of a protective group E to give a compound of the formula VIII. 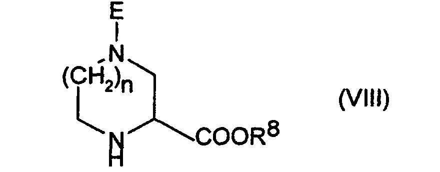 converting the compound of the formula VIII with the sulfonic acid derivative of the formula IV under the abovementioned conditions into a compound of the formula IX 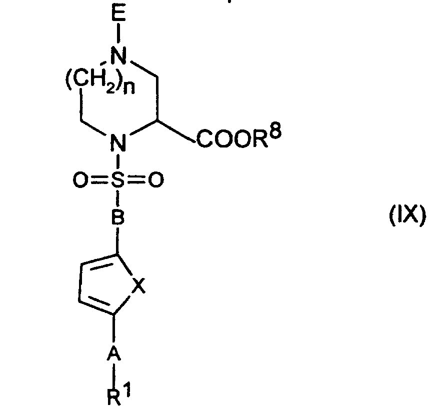 and then converting the compound of the formula IX into the compound of the formula I with removal of the protective group E and of the radical R⁸ with the aid of suitable cleavage reagents or
d) resolving a compound of the formula I, which on account of its chemical structure occurs in enantiomeric forms, prepared by process a), b) or c) into the pure enantiomers by salt formation with enantiomerically pure acids or bases, chromatography on chiral stationary phases or derivatization by means of chiral enantiomerically pure compounds such. as amino acids, separation of the diastereomers thus obtained, and removal of the chiral auxiliary groups, or
e) isolating the compound of the formula I prepared by process a), b), c) or d) either in free form or, in the case of the presence of acidic or basic groups, converting it into physiologically tolerable salts.

6. A pharmaceutical, comprising an efficacious amount of at least one compound of the formula I as claimed in one or more of claims 1 to 4 together with a pharmaceutically suitable and physiologically tolerable excipient, additive and/or other active compounds and auxiliaries.

7. The use of at least one compound of the formula I as claimed in one or more of claims 1 to 4 for the production of pharmaceuticals for the prophylaxis and therapy of disorders in the course of which an increased activity of matrix-degrading metalloproteinases is involved.

8. The use as claimed in claim 7 for the treatment of degenerative joint disorders such as osteoarthroses, spondyloses, chondrolysis after joint trauma or relatively long immobilization of the joint after meniscus or patella injuries or tears of the ligaments, disorders of the connective tissue such as collagenoses, periodontal disorders, wound healing disorders and chronic disorders of the locomotory apparatus such as inflammatory, immunologically or metabolically related acute and chronic arthritides, arthropathies, myalgias and disorders of the bone metabolism, ulceration, atherosclerosis and stenoses, but also for the treatment of inflammations, carcinomatous disorders, formation of tumor metastases, cachexia, anorexia and septic shock.

9. A process for the production of a pharmaceutical which comprises bringing at least one compound of the formula I as claimed in one or more of claims 1 to 4 into a suitable administration form using a pharmaceutically suitable and physiologically tolerable excipient and, if appropriate, other suitable active compounds, additives or auxiliaries.

## Revendications

1. Composé de formule I 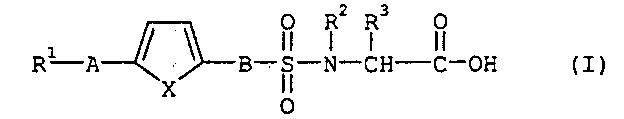 et/ou forme stéréoisomère du composé de formule I, et/ou sel compatible d'un point de vue physiologique du composé de formule I, où
R¹ est :
1. le groupe phényle,
2. un groupe phényle qui est une ou deux fois substitué par les substituants suivants :
2.1 alkyle en C₁-C₆, à chaîne droite ou ramifiée, ou cyclique,
2.2 -OH,
2.3 (alkyle en C₁-C₆)-C(O)-O-,
2.4 (alkyle en C₁-C₆)-O-,
2.5 (alkyle en C₁-C₆)-O-(alkyle en C₁-C₄)-O-,
2.6 halogéno,
2.7 -CF₃,
2.8 -CN,
2.9 -NO₂,
2.10 HO-C(O)-,
2.11 (alkyle en C₁-C₆)-O-C(O)-,
2.12 méthylènedioxo,
2.13 R⁴-(R⁵)N-C(O)- ou
2.14 R⁴-(R⁵)N-, ou
3. un groupe hétéroaromatique choisi dans les groupes 3.1 à 3.15 ci-après, qui est non substitué, ou est substitué comme décrit en 2.1 à 2.14,
3.1 pyrrole,
3.2 pyrazole,
3.3 imidazole,
3.4 triazole,
3.5 thiophène,
3.6 thiazole,
3.7 oxazole,
3.8 isoxazole,
3.9 pyridine,
3.10 pyrimidine,
3.11 indole,
3.12 benzothiophène,
3.13 benzimidazole,
3.14 benzoxazole ou
3.15 benzothiazole,
R², R⁴ et R⁵ sont identiques ou différents et représentent chacun :
1. un atome d'hydrogène, ou un groupe
2. (alkyle en C₁-C₆)-,
3. HO-C(O) - (alkyle en C₁-C₆)-,
4. phényl-(CH₂)ₙ-, où le groupe phényle est non substitué ou une à deux fois substitué comme décrit en 2.1 à 2.14, ou est substitué par un ou plusieurs substituants -NH-C(O)-(alkyle en C₁-C₃), et n représente le nombre entier zéro, 1 ou 2, ou
5. picolyle, ou bien
6. R⁴ et R⁵, avec le groupe amino nucléaire, forment un noyau à 4 à 7 chaînons, dans lequel éventuellement l'un des atomes de carbone est remplacé par -O-, -S-ou -NH-, ou encore deux atomes de carbone voisins du cycle à 4 à 7 chaînons représentent une partie d'un résidu benzyle,
R³ représente un groupe
1. (alcényle en C₂-C₁₀)-, où le groupe alcényle a une chaîne droite ou ramifiée,
2. R²-S(O)ₙ-(alkyle en C₁-C₆)-, où n a les significations données ci-dessus, et R² a les significations données ci-dessus pour 2 à 5,
3. R²-S(O) (=NH) - (alkyle en C₁-C₆)-, où R² a les significations données ci-dessus pour 2 à 5,
4. 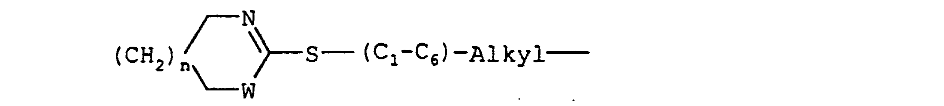 où n est le nombre entier zéro, 1 ou 2, et W est un atome d'azote, d'oxygène ou de soufre,
5. -(CH₂)ₘ-P(O) (OH) - (alkyle en C₁-C₃), où m est le nombre entier zéro, 1, 2, 3, 4, 5 ou 6, ou
6. R⁶-C(O)-(alkyle en C₁-C₆)-, où R⁶ est un groupe
1. (alkyle en C₁-C₆)-,
2. HO-C(O)-(CH(R³)-NH-, où R³ est
2.1. un atome d'hydrogène ou un groupe,
2.2 (alkyle en C₁-C₁₀)-, où le groupe alkyle est non substitué et/ou un atome d'hydrogène du radical alkyle est remplacé par -OH,
2.3. (alcényle en C₂-C₁₀)-, où le groupe alcényle a une chaîne droite ou ramifiée,
2.4. R²-O-(alkyle en C₁-C₆)-, où R² a les significations données ci-dessus pour 1. à 5.,
2.5. R²-S(O)ₙ-(alkyle en C₁-C₆)-, où n est le nombre entier zéro, 1 ou 2, et R² a les significations données ci-dessus pour 1. à 5.,
2.6. R²-S(O) (=NH) - (alkyle en C₁-C₆)-, où R² a les significations données ci-dessus pour 1. à 5.,
2.7 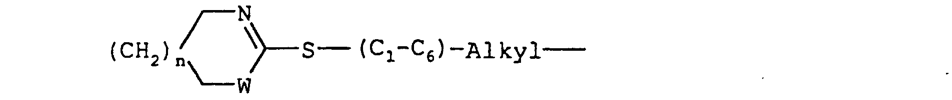 où n est le nombre entier zéro, 1 ou 2, et W est un atome d'azote, d'oxygène ou de soufre,
2.8. phényl-(CH₂)ₘ-, où m est le nombre entier zéro, 1, 2, 3, 4, 5 ou 6, et/ou un atome d'hydrogène de la chaîne -(CH₂)ₘ- est remplacé par -OH, et le groupe phényle est non substitué ou une ou deux fois substitué
2.8.1. comme décrit ci-dessus en 2.1. à 2.14., ou par
2.8.2. des groupes -O-(CH₂)ₘ-phényle où le groupe phényle est non substitué ou est une deux fois substitué comme décrit ci-dessus en 2.1. à 2.14., et m est le nombre entier zéro, 1, 2, 3, 4, 5 ou 6, ou
2.8.3. des groupes -C(O)-(CH₂)ₘ-phényle, où le groupe phényle et m sont tels que définis en 2.8.2,
2.9. hétéroaryl-(CH₂)ₘ-, où le groupe hétéroaryle est tel que défini en 3.1 à 3.15, m est le nombre entier zéro, 1, 2, 3, 4, 5 ou 6 comme défini ci-dessus, et/ou un atome d'hydrogène de la chaîne -(CH₂)ₘ- est remplacé par -OH, et le groupe hétéroaryle est non substitué ou une à deux fois substitué
2.9.1. comme décrit ci-dessus en 2.1. à 2.14., ou par des groupes
2.9.2. -CH(O),
2.9.3. -SO₂-phényle, où le groupe phényle est non substitué ou est tel que défini en 2.8.2. à 2.8.3., ou
2.9.4. -O- (CH₂)ₘ-phényle, où m est le nombre entier zéro, 1, 2, 3, 4, 5 ou 6, ou
2.10. -(CH₂)ₘ-P(O)(OH)-(alkyle en C₁-C₃), où m est le nombre entier zéro, 1, 2, 3, 4, 5 ou 6,
3. phényle, où le groupe phényle est non substitué, ou est substitué comme décrit en.2.1. à 2.14.,
4. hétéroaryle, où le groupe hétéroaryle est tel que défini en 3.1. à 3.15. et/ou est substitué comme défini en 2.1. à 2.14.,
5. R⁴-(R⁵)N-NH-, où R⁴ et R⁵ sont tels que définis ci-dessus,
6. R²O-, où R² a les significations données ci-dessus pour 2 à 5,
7. R⁴-(R⁵)N-, où R⁴ et R⁵ ont les significations données ci-dessus pour 1. à 5., mais ne représentent pas simultanément des atomes d'hydrogène, ou bien
8. hétéroaryl-(CH₂)ₘ-NH-, où le groupe hétéroaryle est tel que défini en 3.1. à 3.15. et/ou est substitué comme décrit en 2.1. à 2.14., et m est le nombre entier zéro, 1, 2, 3, 4, 5 ou 6 ou est substitué par des substituants-(alkyle en C₁-C₄)-COOH, ou bien
R² et R³ forment ensemble un cycle avec un groupe carboxyle nucléaire, de formule partielle II 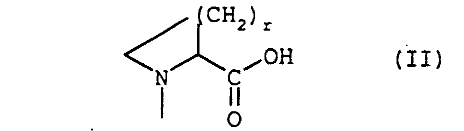 dans laquelle r est le nombre entier zéro, 1, 2 ou 3 et/ou l'un des atomes de carbone du noyau est remplacé par -O-, -S- ou -(R⁷)N-, où R⁷ est
1. un atome d'hydrogène, ou un groupe,
2. alkyle en C₁-C₆,
3. phényle, où le groupe phényle est non substitué ou est substitué comme décrit en 2.1. à 2.14.,
4. benzyle, où le groupe benzyle est non substitué ou est substitué comme décrit en 2.1. à 2.14., ou
5. R²N-C(=NH)-, où R² a les significations données ci-dessus pour 1. à 5.,
et/ou les atomes de carbone du noyau de formule partielle II sont une ou plusieurs fois substitués par des substituants (alkyle en C₁-C₆)-, phényl-, phényl-(CH₂)ₘ- ou -OH, où m est le nombre entier zéro, 1, 2, 3, 4, 5 ou 6,
A est
a) une liaison covalente,
b) -O-,
c) -CH=CH-, ou
d) -C≡C-,
B est
a) -(CH₂)ₘ-, où m est le nombre entier zéro, 1, 2, 3, 4, 5 ou 6,
b) -O-(CH₂)ₚ, où p est un nombre entier de 1 à 5, ou
c) -CH=CH-, et
X est -CH=CH-, un atome d'oxygène ou un atome de soufre.

2. Composé de formule I selon la revendication 1, **caractérisé en ce que** R¹ est un groupe
1. phényle, ou
2. phényle, qui est une fois substitué par l'un des substituants suivants
2.1 alkyle en C₁-C₆, à chaîne droite ou ramifiée, ou cyclique,
2.2 -OH,
2.3 (alkyle en C₁-C₆)-C(O)-O-,
2.4 (alkyle en C₁-C₆)-O-,
2.5 (alkyle en C₁-C₆)-O-(alkyle en C₁-C₄)-O-,
2.6 halogéno,
2.7 -CF₃, ou
2.8 R⁴-(R⁵)N-,
R²; R⁴ et R⁵ sont identiques ou différents et représentent chacun :
1. un atome d'hydrogène, ou un groupe
2. (alkyle en C₁-C₆)-,
R³ est un groupe
1. R²-S(O)ₙ-(alkyle en C₁-C₆)-, où R² est un groupe (alkyle en C₁-C₆)- ou phényl-(CH₂)ₙ-, et n est le nombre entier zéro ou 1, ou
2. R⁶-C(O)-(alkyle en C₁-C₆)-, où
R⁶ est
1. R²O-, où R² est un atome d'hydrogène ou un groupe alkyle en C₁-C₆, ou
2. R⁴-(R⁵)N-, où R⁴ et R⁵ ont les significations données ci-dessus pour 1. à 2., mais ne représentent pas simultanément des atomes d'hydrogène, ou
R⁴ et R⁵, avec le groupe amino nucléaire, forment un noyau à 5 ou 6 chaînons, dans lequel éventuellement l'un des atomes de carbone est remplacé par -O-, -S- ou -NH-, ou encore deux atomes de carbone voisins du noyau à 5 ou 6 chaînons font partie d'un résidu benzyle, ou
R² et R³ forment ensemble un noyau avec le groupe carboxyle nucléaire de la formule partielle II, où n est le nombre entier 1 ou 2 et/ou l'un des atomes de carbone du noyau est remplacé par -O- ou -(R⁷)N-, et
R⁷ est
1. un atome d'hydrogène, ou un groupe,
2. alkyle en C₁-C₆,
3. phényle, où le groupe phényle est non substitué ou est substitué comme décrit en 2.1. à 2.14.,
4. benzyle, où le groupe benzyle est non substitué ou est substitué comme décrit en 2.1. à 2.14., ou
5. R²N-C(=NH)-, où R² est un groupe (alkyle en C₁-C₆)-, et/ou les atomes de carbone du noyau de formule partielle II sont une fois substitués par un substituant phényle ou -OH,
A est
a) une liaison covalente, ou
b) -O-,
B est
a) -(CH₂)ₘ-, où m est le nombre entier zéro, 1 ou 2, ou
b) -O-(CH₂)ₚ, où p est un nombre entier valant 1 ou 2, et
X est -CH=CH.

3. Composé de formule I selon la revendication 1, **caractérisé en ce que** R¹ est un groupe
1. phényle, ou
2. phényle, qui est une fois substitué par un groupe
2.1. halogéno, en particulier chloro ou fluoro, ou
2.2 R⁴-(R⁵)N-, où R⁴ et R⁵ sont identiques ou différents et représentent chacun un groupe
2.2.1. alkyle en C₁-C₃, ou encore
2.2.2. R⁴ et R⁵ forment avec le groupe amino nucléaire un noyau à 5-6 chaînons, l'un des atomes de carbone étant éventuellement remplacé par -O- ou -N-,
R² est un atome d'hydrogène,
R³ est
1. R⁶-C(O)-(alkyle en C₂-C₃)-, où
R⁶ représente
1. R⁴-(R⁵)N-, où R⁴ et R⁵ sont identiques ou différents mais ne représentent pas simultanément des atomes d'hydrogène, et représentent chacun
1.1. un atome d'hydrogène ou un groupe
1.2. (alkyle en C₁-C₃)-,
1.3. phényl-(CH₂)ₙ-, où le groupe phényle est non substitué ou est une ou deux fois substitué comme décrit en 2.1 à 2.14, et n est le nombre entier zéro, 1 ou 2,
1.4. R⁴ et R⁵ forment ensemble avec le groupe amino nucléaire un noyau à 5-6 chaînons, l'un des atomes de carbone étant éventuellement remplacé par -O- ou -NH-, ou encore forment un radical indoline, ou
1.5. HO-C(O)-CH(R³)-NH-, où R³ est un groupe hétéro-aryle-(CH₂)ₘ-, où le groupe hétéroaryle est défini comme ci-dessus en 3.5, 3.11 ou 3.13, et le groupe hétéroaryle est non substitué ou une fois substitué comme décrit en 2.1 ou 2.14, et m est le nombre entier 1 ou 2,
A est une liaison covalente,
B est -(CH₂)ₒ-, où o désigne zéro, et
X est -CH=CH-.

4. Composé de formule VI 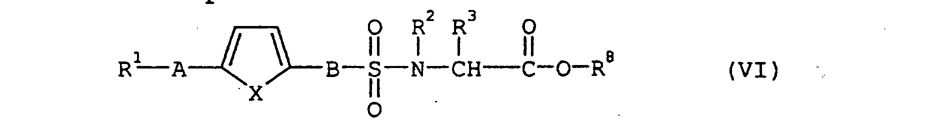 et/ou forme stéréoisomère du composé de formule VI et/ou sel compatible d'un point de vue physiologique du composé de formule VI, où R¹, A, X, B, R² et R³ ont les significations données dans le composé de formule I selon la revendication 1, et R⁸ est un atome d'hydrogène ou un groupe alkyle en C₁-C₆, phényle ou benzyle.

5. Procédé de préparation du composé de formule I selon l'une ou plusieurs des revendications 1 à 4, **caractérisé en ce que**
a) on fait réagir un acide aminocarboxylique de formule III, 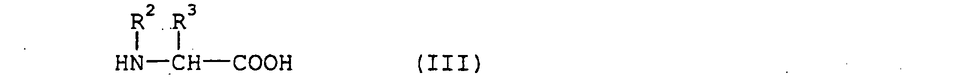 dans laquelle R² et R³ sont tels que définis dans la formule I, avec un dérivé d'acide sulfonique de formule IV, 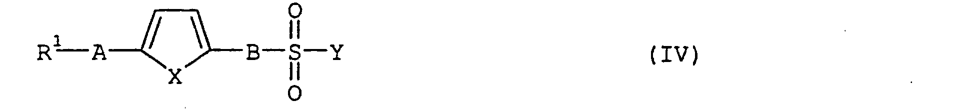 dans laquelle R¹, A et B sont tels que définis dans la formule I, et Y est un atome d'halogène, un groupe imidazolyle ou -OR⁸, où R⁸ est un atome d'hydrogène ou un groupe alkyle en C₁-C₆, phényle ou benzyle, éventuellement substitué,
en présence d'une base ou éventuellement d'un agent déshydratant, pour obtenir un composé de formule I, ou bien
b) on fait réagir un ester d'acide aminocarboxylique de formule V, 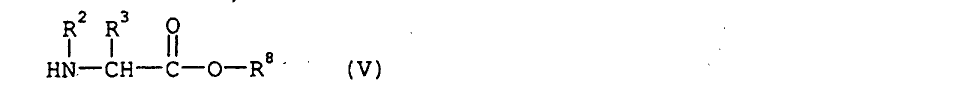 dans laquelle R², R³ et R⁸ ont les significations données ci-dessus, avec un dérivé d'acide sulfonique de formule IV dans les conditions données ci-dessus, pour obtenir un composé de formule VI 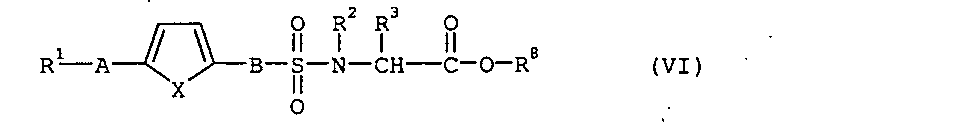 et on convertit en un composé de formule I le composé de formule VI avec élimination du résidu R⁸, de préférence en présence d'une base ou d'un acide, ou bien
c) on fait réagir le composé de formule VII 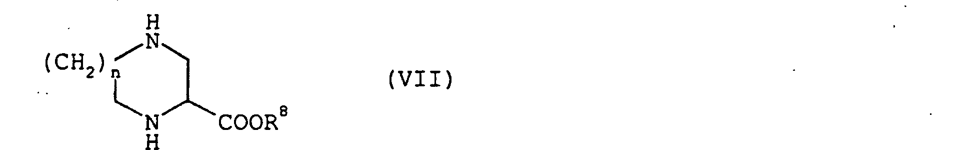 dans laquelle n est le nombre entier zéro, 1 ou 2,
à l'aide d'un groupe protecteur E pour obtenir un composé de formule VIII 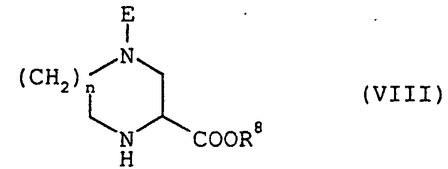 on convertit le composé de formule VIII avec le dérivé d'acide sulfonique de formule IV et dans les conditions indiquées ci-dessus en un composé de formule IX 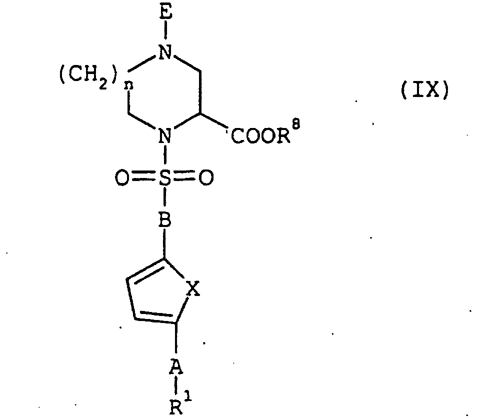 puis on convertit le composé de formule IX, avec élimination du groupe protecteur E et du résidu R⁸, et à l'aide de réactifs de dissociation appropriés, en le composé de formule I, ou bien
d) on sépare en les énantiomères purs un composé de formule I préparé par les procédés a), b) ou c), qui en raison de sa structure chimique se présente sous des formes énantiomères, par salification avec des acides ou des bases ayant une pureté énantiomère, chromatographie sur des bases stationnaires chirales ou dérivatisation à l'aide de composés chiraux à pureté énantiomère tels que des acides aminés, séparation des diastéréoisomères ainsi obtenus, et élimination des groupes auxiliaires chiraux, ou bien
e) on isole sous forme libre le composé de formule I préparé par les procédés a), b), c) ou d), ou encore, en présence de groupes acides ou basiques, on le convertit en des sels acceptables d'un point de vue physiologique.

6. Médicament, **caractérisé en ce qu'**il contient une quantité efficace d'au moins un composé de formule I selon l'une ou plusieurs des revendications 1 à 4, en même temps qu'un excipient, un additif et/ou d'autres principes actifs et adjuvants acceptables d'un point de vue pharma-ceutique et physiologique.

7. Utilisation d'au moins un composé de formule I selon l'une ou plusieurs des revendications 1 à 4 pour préparer des médicaments destinés à la prophylaxie et à la thérapie de maladies à l'évolution desquelles contribue une activité renforcée de métalloprotéinases qui dégra-dent la matrice.

8. Utilisation selon la revendication 7 pour le traitement des maladies dégénératives des articulations telles que les ostéoarthroses, les spondyloses, les atrophies du cartilage après un traumatisme articulaire ou une immobilisation de longue durée des articulations après des lésions du ménisque ou de la rotule ou une déchirure ligamentaire, les maladies du tissu conjonctif telles que les collagénoses, les maladies périodontiques, les trou-bles de la cicatrisation et les maladies chroniques de l'appareil moteur tels que les arthrites aiguës ou chro-niques inflammatoires, immunologiques ou d'origine méta-bolique, les arthropathies, les myalgies et les troubles du métabolisme osseux, l'ulcération, l'athérosclérose et les sténoses, mais aussi pour le traitement des inflamma-tions, des maladies cancéreuses, de la formation de méta-stases tumorales, de la cachexie, de l'anorexie et du choc septique.

9. Procédé de préparation d'un médicament, **caractérisé en ce qu'**on met sous une forme pharmaceutique appropriée au moins un composé de formule I selon l'une ou plusieurs des revendications 1 à 4 avec un excipient acceptables d'un point de vue pharmaceutique et toléré du point de vue physiologique, et éventuellement d'autres principes actifs, additifs et adjuvants appropriés.
